# EUROPEAN PATENT APPLICATION

(11) **EP 4 545 972 A1**
(43) Date of publication of application: **30.04.2025**
(21) Application number: 22947594.2
(22) Date of filing: 23.08.2022
(51) Int. Cl.: G01N 33/68, G01N 33/541, G01N 33/58, G01N 21/64

(54) **REAGENT COMBINATION, KIT, SYSTEM, AND METHOD FOR DETECTING TARGET PROTEIN**

(30) Priority: 23.06.2022 CN 202210718616
(71) Applicant: Nanjing Poclight Biotechnology Co., Ltd, Nanjing, Jiangsu 210061 (CN)
(72) Inventor: CAO, Dan, Nanjing, Jiangsu 210061 (CN); CHENG, Shun, Nanjing, Jiangsu 210061 (CN)
(74) Representative: Murgitroyd & Company
(86) International application number: PCT/CN2022/114272
(87) International publication number: WO 2023/245853

(57) **Abstract**

A reagent combination, kit, system, and method for detecting a target protein are disclosed. In a case that a target protein is contained in the solution to be detected, a first antibody and a second antibody are used to form a double-antibody sandwich structure with the target protein. Through the complementary pairing between multiple single stranded DNAs, the donor fluorescent molecules excite the acceptor fluorescent molecules to emit fluorescence, so as to calculate the content of the target protein. The detection method in the present dsiclosure is simple, has little background interference, high sensitivity and small measurement error.

## Description

### CROSS-REFERENCE TO RELATED APPLICATION

The present disclosure claims priority of Chinese Patent Application No. 202210718616.7, filed on June 23, 2022, and entitled "Reagent composition, Kit, System, and Method for Detecting Target Protein", the disclosure of which is incorporated herein by reference in its entirety.

### TECHNICAL FIELD

The present disclosure relates to the technical field of chemiluminescence detection, and in particularly, to a reagent composition, a kit, a system, and a method for detecting a target protein.

### BACKGROUND

A chemiluminescence immunoassay (CLIA) can be used to detect whether a target protein exists in a solution to be detected. In this method, a luminescent group is connected to an antibody against the target protein to produce a detection probe. In a case that the target protein is not present in the solution to be detected, the antibody in the detection probe cannot specifically bind to the target protein. At this time, the luminescent group generally has no luminescent activity and will not generate strong fluorescence intensity. In a case that the target protein is present in the solution to be detected, the antibody in the detection probe can specifically bind to the target protein. At this time, the luminescent group emits strong fluorescence under an action of a luminescent substrate. A chemiluminescence detection instrument can be used to obtain the fluorescence intensity. Since the value of the fluorescence intensity has a function relationship with the content of the target protein in the solution to be detected, the concentration of the target protein in the solution to be detected can be calculated according to the value of the fluorescence intensity.

However, in the above method, a single fluorescent group is used to emit fluorescence, and the content of the target protein is calculated according to the fluorescence intensity of the single fluorescent group. Since a single fluorescent group will emit background fluorescence in the absence of the target protein, even if there is a control reagent, the background fluorescence will affect the true value of the fluorescence intensity, thereby causing a large error when detecting the content of the target protein.

### SUMMARY

Some embodiments of the present disclosure aim to provide a reagent composition, a kit, a system, and a method for detecting a target protein, which can solve the problem that background fluorescence emitted by a single fluorescent group in the existing chemiluminescence detection method interferes with the actual content of the target protein. Some embodiments mentioned herein may come from the same embodiment or from different embodiments.

In order to solve the above technical problems, some embodiments of the present disclosure provide a reagent composition for detecting a target protein. The reagent composition includes at least a first detection probe, a second detection probe, a third detection probe, and a fourth detection probe.

The first detection probe is formed by coupling at least a first single stranded DNA and a first antibody. The first single stranded DNA includes a first pairing sequence and a second pairing sequence. The first pairing sequence and the second pairing sequence are not directly adjacent, but are separated by two or five nucleotides. The first antibody is capable of specifically binding to a first epitope of the target protein. In the present disclosure, single stranded DNA is described in a manner from left to right from from 5' end to 3' end.

The second detection probe is formed by coupling at least a second antibody, a second single stranded DNA, and an acceptor fluorescent molecule in sequence. The second single stranded DNA has a third pairing sequence and a fourth pairing sequence. The third pairing sequence is complementary to the second pairing sequence. The third pairing sequence is directly connected to the fourth pairing sequence without intervening nucleotides. The second antibody is capable of specifically binding to a second epitope of the target protein. The first epitope is different from the second epitope, so that the first antibody, the second antibody and the target protein can form a stable double-antibody sandwich structure.

The third detection probe is formed by coupling at least a donor fluorescent molecule and a third single stranded DNA. The third single stranded DNA includes a fifth pairing sequence and a sixth pairing sequence. The fifth pairing sequence is directly connected to the sixth pairing sequence without intervening nucleotides. The fifth pairing sequence is complementary to the fourth pairing sequence, and the sixth pairing sequence is complementary to the first pairing sequence. Through complementary pairing between the single stranded DNA molecules described above, the first single stranded DNA, the second single stranded DNA, and the third single stranded DNA can form a stem-loop structure. The structure of the "stem" is ⊥-shaped, and the paired areas show a double helix structure. The donor fluorescent molecules emit a first fluorescence when oxidized by an oxidant and in the absence of an antioxidant. The first fluorescence, as an excitation light, excites the acceptor fluorescent molecules to emit a second fluorescence based on the fluorescence resonance energy transfer effect under a condition that the first single stranded DNA, the second single stranded DNA and the third single stranded DNA are paired with each other, so that the content of the target protein can be obtained according to the intensity of the second fluorescence. In a case that the intensity of the second fluorescence is not obtained, the content of the target protein is zero, indicating that there is no target protein at this time. The relationship between the intensity of the second fluorescence and the content of the target protein can be obtained in advance, so that a correlation between the intensity of the second fluorescence and the content of the target protein can be established. Therefore, the measurement of the content of the target protein can be converted into the measurement of the value of the fluorescence intensity.

The fourth detection probe includes an antioxidant. The antioxidant can inhibit the donor fluorescent molecule from being oxidized to emit first fluorescence. The donor fluorescent molecules in each embodiment of the present disclosure emit light by oxidation instead of being irradiated by excitation light. Therefore, it is necessary to prevent the donor fluorescent molecules from being oxidized by oxides in the solution to be detected to generate background fluorescence before the first detection probe, the second detection probe, and the third detection probe form a stable structure, which is a kind of noise and affects the actual value of the fluorescence measurement, thus affecting the accuracy of the measurement results of the target protein content. The antioxidant and the oxidant are not present simultaneously in the sample to be detected, so as not to neutralize the oxidizing and luminescence of the oxidant on the donor fluorescent molecules by the antioxidant. Therefore, it is necessary to remove the antioxidant before adding the oxidant.

In some embodiments of the present disclosure, first single stranded DNA, the second single stranded DNA, and the third single stranded DNA are complementary and paired in the presence of the target protein to form a stem-loop structure, so that a distance between the donor fluorescent molecule and the acceptor fluorescent molecule is less than a limit distance at which fluorescence resonance energy transfer can occur, for example, in the range of 70 angstroms to 99 angstroms, or in the range of 7 nm to 10 nm. As a result, a fluorescence resonance energy transfer effect can occur between the donor fluorescent molecules and the acceptor fluorescent molecules.

In some embodiments of the present disclosure, the donor fluorescent molecules are acridinium esters, and the acceptor fluorescent molecules are quantum dots.

In some embodiments of the present disclosure, the acridinium ester has a maximum emission wavelength of 430 nm.

The maximum absorption wavelength of the acceptor fluorescent molecules may be in the range of 420 nm to 520 nm, for example, it may be 470 nm. The maximum emission wavelength of the acceptor fluorescent molecules may be in the range of 595 nm to 615 nm, for example, it may be 605 nm. In some embodiments of the present disclosure, the quantum dots are core-shell quantum dots having a core layer material selected from one or more of CdSe, CdS, CdTe, CdSeTe, CdZnS, ZnTe, CdSeS, PbS, and PbTe, and a shell layer material selected from one or more of ZnS, ZnSe, ZnSeS, PbS, and PbSeS.

In some embodiments of the present disclosure, the particle size of the quantum dots may range from 3 nm to 5 nm, or may range from 4.1 nm to 4.2 nm.

In some embodiments of the present disclosure, a ribose ring at 3' end of the first single stranded DNA is covalently linked to an amino group of the first antibody through a first coupling agent. The ribose ring at the 5' end of the first single stranded DNA is not modified. Optionally, the ribose ring at the 3' end of the first single stranded DNA is modified by a NH2C7 modifying group, and the NH2C7 modifying group is covalently linked to the amino group of the first antibody through the first coupling agent. Optionally, the first coupling agent is suberate bis(sulfosuccinimidyl) sodium salt.

In some embodiments of the present disclosure, the 3' end of the second single stranded DNA is linked to an acceptor fluorescent molecule. Optionally, the ribose ring at the 3' end of the second single stranded DNA is modified by a sulfhydryl group, a surface of the acceptor fluorescent molecule is modified by an amino group, and the sulfhydryl group is covalently linked to the amino group on the surface of the acceptor fluorescent molecule through a third coupling agent. Optionally, the third coupling agent is 4-(N-maleimidomethyl)cyclohexane-1-carboxylic acid N-hydroxysuccinimide ester.

In some embodiments of the present disclosure, the ribose ring at the 5' end of the second single stranded DNA is covalently linked to the amino group of the second antibody through a second coupling agent. Optionally, the ribose ring at the 5' end of the second single stranded DNA is modified by a NH2C6 modifying group, and the NH2C6 modifying group is covalently linked to the amino group of the second antibody through a second coupling agent. Optionally, the second coupling agent is suberate bis(sulfosuccinimidyl) sodium salt.

In some embodiments of the present disclosure, the 5' end of the third single stranded DNA is covalently linked to a donor fluorescent molecule. Optionally, the ribose ring at the 5' end of the third single stranded DNA is modified by a NH2C6 modifying group, and the NH2C6 modifying group is covalently linked to the donor fluorescent molecule.

In some embodiments of the present disclosure, in the first single stranded DNA, the first pairing sequence is located upstream of the second pairing sequence in an orientation from 5' end to 3' end. In the second single stranded DNA, the third pairing sequence is located upstream of the fourth pairing sequence in an orientation from 5' end to 3' end. In the third single stranded DNA, the fifth pairing sequence is located upstream of the sixth pairing sequence in an orientation from 5' end to 3' end.

In some embodiments of the present disclosure, the first single stranded DNA has 55 nucleotides, the first pairing sequence covers the 3rd to 10th base sites of the first single stranded DNA starting from the 5' end, and the second pairing sequence covers the 13th to 19th base sites of the first single stranded DNA starting from the 5' end. The second single stranded DNA has 53 nucleotides, the third pairing sequence covers 37th to 43rd base sites starting from the 5' end of the second single stranded DNA, and the fourth pairing sequence covers 44th to 51th base sites starting from the 5' end of the second single stranded DNA. The third single stranded DNA has 22 nucleotides, the fifth pairing sequence covers the 3rd to 10th base sites of the third single stranded DNA starting from the 5' end, and the sixth pairing sequence covers the 11th to 18th base sites of the third single stranded DNA starting from the 5' end. The complementary pairing between the six pairing sequences enables the first single stranded DNA, the second single stranded DNA, and the third single stranded DNA to form a stem-loop structure, and the "stem" is ⊥-shaped.

In some embodiments of the present disclosure, the first pairing sequence is GCTGAGTT from the 5' end to 3' end, and the sixth pairing sequence is AACTCAGC from the 5' end to 3' end. The second pairing sequence is CAACGAC from the 5' end to 3' end, and the third pairing sequence is GTCGTTG from the 5' end to 3' end. The fourth pairing sequence is GCTGAGAT from the 5' end to 3' end, and the fifth pairing sequence is ATCTCAGC from the 5' end to 3' end. Two pairing sequences within the same single stranded DNA are not paired with each other, but with another pairing sequence of the single stranded DNA.

In some embodiments of the present disclosure, the full-length sequence of the first single stranded DNA is shown in SEQ ID No: 1, the full-length sequence of the second single stranded DNA is shown in SEQ ID No: 2, and the full-length sequence of the third single stranded DNA is shown in SEQ ID No: 3.

In some embodiments of the present disclosure, G in the first single stranded DNA, the second single stranded DNA, and/or the third single stranded DNA may be replaced by ᵢₛₒG, and C in the first single stranded DNA, the second single stranded DNA, and/or the third single stranded DNA may be replaced by ᵢₛₒC. Both ᵢₛₒG and ᵢₛₒC are non-natural base pairs, which can replace the natural bases G and C, respectively. The use of non-natural base pairs for pairing can effectively avoid mismatching between the first single stranded DNA, the second single stranded DNA, and/or the third single stranded DNA and the natural nucleic acids in the solution to be detected, thereby avoiding the mismatch affecting the formation of the stem-loop structure and further avoiding measurement errors caused by the mismatch.

ᵢₛₒG has a structural formula of: 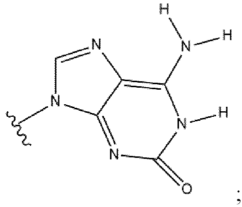 and
ᵢₛₒC has a structural formula of:

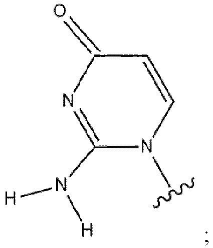

A bonding form of ᵢₛₒG and ᵢₛₒC is as follows: 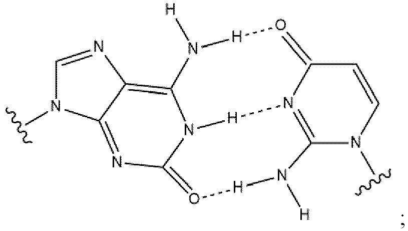 wherein 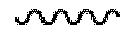 indicates a site connecting to deoxyribose in a DNA molecule.

In some embodiments of the present disclosure, G in both the first pairing sequence and the sixth pairing sequence is replaced by ᵢₛₒG, and C in both the first pairing sequence and the sixth pairing sequence is replaced by ᵢₛₒC. The first pairing sequence is ᵢₛₒGᵢₛₒCTᵢₛₒGAᵢₛₒGTT from the 5' end to 3' end, and the sixth pairing sequence is AAᵢₛₒCTᵢₛₒCAᵢₛₒGᵢₛₒC from the 5' end to 3' end.

In some embodiments of the present disclosure, G in both the second pairing sequence and the third pairing sequence is replaced by ᵢₛₒG, and C in both the second pairing sequence and the third pairing sequence is replaced by ᵢₛₒC. The second pairing sequence is ᵢₛₒCAAᵢₛₒCᵢₛₒGAᵢₛₒC from the 5' end to 3' end, and the third pairing sequence is ᵢₛₒGTᵢₛₒCᵢₛₒGTTᵢₛₒG from the 5' end to 3' end.

In some embodiments of the present disclosure, G in both the fourth pairing sequence and the fifth pairing sequence is replaced by ᵢₛₒG, and C in both the fourth pairing sequence and the fifth pairing sequence is replaced by ᵢₛₒC. The fourth pairing sequence is ᵢₛₒGᵢₛₒCTᵢₛₒGAᵢₛₒGAT from the 5' end to 3' end, and the fifth pairing sequence is ATᵢₛₒCTᵢₛₒCAᵢₛₒGᵢₛₒC from the 5' end to 3' end.

In some embodiments of the present disclosure, the full-length sequence of the first single stranded DNA is as follows:
AᵢₛₒCᵢₛₒGᵢₛₒCTᵢₛₒGAᵢₛₒGTTATᵢₛₒCAAᵢₛₒCᵢₛₒGAᵢₛₒCTTTTTTTATᵢₛₒCAᵢₛₒCATᵢₛₒCAᵢₛₒGᵢₛₒGᵢₛₒCTᵢₛₒCTAᵢₛₒGᵢₛₒ CᵢₛₒGTATᵢₛₒGᵢₛₒCTATTᵢₛₒG. In other embodiments of the present disclosure, the full-length sequence of the first single stranded DNA is a sequence in which at least a part or all of G is replaced by ᵢₛₒG, and at least a part or all of C is replaced by ᵢₛₒC in the sequence shown in SEQ ID No: 1.

In some embodiments of the present disclosure, the full-length sequence of the second single stranded DNA is as follows:
TAᵢₛₒCᵢₛₒGTᵢₛₒCᵢₛₒCAᵢₛₒGAAᵢₛₒCTTTAᵢₛₒCᵢₛₒCAAAᵢₛₒCᵢₛₒCAᵢₛₒCAᵢₛₒCᵢₛₒCᵢₛₒCTTTTTTTᵢₛₒGTᵢₛₒCᵢₛₒGTTᵢₛₒG_{i so}GᵢₛₒCTᵢₛₒGAᵢₛₒGATTᵢₛₒC. In other embodiments of the present disclosure, the full-length sequence of the second single stranded DNA is a sequence in which at least a part or all of G is replaced by ᵢₛₒG, and at least a part or all of C is replaced by ᵢₛₒC in the sequence shown in SEQ ID No: 2.

In some embodiments of the present disclosure, the full-length sequence of the third single stranded DNA is as follows:
ᵢₛₒCᵢₛₒGATᵢₛₒCTᵢₛₒCAᵢₛₒGᵢₛₒCAAᵢₛₒCTᵢₛₒCAᵢₛₒGᵢₛₒCAᵢₛₒGᵢₛₒCᵢₛₒG. In other embodiments of the present disclosure, the full-length sequence of the third single stranded DNA is a sequence in which at least a part or all of G is replaced by ᵢₛₒG, and at least a part or all of C is replaced by ᵢₛₒC in the sequence shown as SEQ ID No: 3.

In some embodiments of the present disclosure, the fourth detection probe further comprises a carrier molecule whose surface binds to the antioxidant. The function of the antioxidant is mainly to prevent the donor fluorescent molecules from being oxidized, thereby producing background fluorescence. The antioxidant is selected from any one or more of cannabidiol, vitamin C, vitamin E, tea polyphenol and glutathione. In some embodiments of the present disclosure, the oxidizing agent includes an alkaline solution of hydrogen peroxide.

In some embodiments of the present disclosure, the carrier molecule is graphene oxide. The carboxyl group on the graphene oxide is bonded to a hydroxyl group on the antioxidant through a sulfoxide condensing agent, and the carboxyl group on the graphene oxide is bonded to an amino group on the antioxidant through 1-(3-dimethylaminopropyl)-3-ethylcarbodiimide hydrochloride. Since the antioxidant and oxidant cannot be added to the solution to be detected at the same time, otherwise, the oxidant cannot oxidize the donor fluorescent molecules to emit light, in this case, it is necessary to remove the antioxidant from the solution to be detected before adding the oxidant. It is more beneficial to remove antioxidants by binding antioxidants to graphene oxide carriers.

In various embodiments of the present disclosure, fluorescence resonance energy transfer between two fluorescent groups is used to generate the second fluorescence to be measured. The donor fluorescent molecules emit light by oxidation, and the acceptor fluorescent molecules emit light by photoexcitation. The first fluorescence emitted by the donor fluorescent molecules can excite the acceptor fluorescent molecules to emit the second fluorescence, and the content of the target protein can be obtained only by collecting the second fluorescence. This detection method eliminates the influence of background fluorescence of donor fluorescent group on the measurement results, and improves the sensitivity of detection.

Some embodiments of the present disclosure also provide a method for detecting a target protein, which includes steps as follows:
(1) Providing a first detection probe, a second detection probe, a third detection probe, and a fourth detection probe; adding the first detection probe, the second detection probe, the third detection probe, and the fourth detection probe into a solution to be detected, and mixing to form a sample to be detected. The first antibody is capable of specifically binding to a first epitope of the target protein. The second detection probe is formed by coupling at least a second antibody, a second single stranded DNA, and an acceptor fluorescent molecule in sequence. The second single stranded DNA has a third pairing sequence and a fourth pairing sequence, and the third pairing sequence is complementary to the second pairing sequence. The second antibody is capable of specifically binding to a second epitope of the target protein. The third detection probe is formed by coupling at least a donor fluorescent molecule and a third single stranded DNA. The third single stranded DNA includes a fifth pairing sequence and a sixth pairing sequence, the fifth pairing sequence is complementary to the fourth pairing sequence, and the sixth pairing sequence is complementary to the first pairing sequence. Under a condition that the target protein is contained in the solution to be detected, the first antibody, the target protein and the second antibody form a double-antibody sandwich structure, the first single stranded DNA, the second single stranded DNA and the third single stranded DNA form a stem-loop structure, and the donor fluorescent molecules and the acceptor fluorescent molecules are located on the same side of the stem-loop structure, so that fluorescence resonance energy transfer can occur between the two fluorescent molecules.
(2) Removing the fourth detection probe for inhibiting donor fluorescent molecules from being oxidized to emit fluorescence from the sample to be detected, adding an oxidant for oxidizing the donor fluorescent molecules to emit a first fluorescence, and collecting a second fluorescence at a maximum emission wavelength of the acceptor fluorescent molecules, thus excluding the fluorescence at wavelengths other than the maximum emission wavelength.
(3) Determining that the target protein is not contained in the solution to be detected in a case that the second fluorescence is not collected; and obtaining a content of the target protein in the solution to be detected according to an intensity of the second fluorescence based on a functional relationship between fluorescence intensity of and protein content in a case that the second fluorescence is collected.

When collecting the intensity of the second fluorescence, in order to avoid interference of the first fluorescence on the second fluorescence, a filter may be used to filter out the fluorescence produced by the oxidation of the donor fluorescent molecules, and only the second fluorescence is allowed to pass through the filter, thereby collecting the second fluorescence emitted by the acceptor fluorescent molecules, and obtaining the content of the target protein according to the intensity of the second fluorescence. The solution to be detected can come from a blood sample. The composition of the blood is complex and contains a plurality of oxidizing substances. If the donor fluorescent molecules are oxidized by these oxidizing substances, background fluorescence will be produced, thereby affecting the accuracy of the measurement results. Therefore, in order to reduce the effect of background fluorescence on the measurement results, in the present disclosure, the first fluorescence emitted by the donor fluorescent groups is not used, instead, the second fluorescence emitted by the acceptor fluorescent groups is used as the detection fluorescence signal.

In some embodiments of the present disclosure, the working concentration of the first detection probe in the sample to be detected may be 1 nM to 20 nM. The working concentration of the second detection probe may be 1 nM to 20 nM. The working concentration of the third detection probe may be 0.05 nM to 0.2 nM. The working concentration of the fourth detection probe may be 15 µg/ml to 25 µg/ml.

In some embodiments of the present disclosure, the solution to be detected is from a blood sample such as a whole blood sample, a serum sample, or a plasma sample.

In some embodiments of the present disclosure, the time for mixing may be from 5 minutes to 10 minutes.

In some embodiments of the present disclosure, the temperature for mixing may be from 36 °C to 37 °C.

In some embodiments of the present disclosure, the volume of the oxidant may be 200 µL and the oxidant is an alkaline hydrogen peroxide solution with a pH of 8.0. The alkaline hydrogen peroxide solution is obtained by dissolving hydrogen peroxide in TBS buffer, wherein the final concentration of hydrogen peroxide is 0.1 M, and the final concentration of TBS is 10 mM.

In some embodiments of the present disclosure, the target protein includes troponin, procalcitonin, or thyroid stimulating hormone.

In some embodiments of the present disclosure, in the mixed sample to be detected, the antibodies in the first detection probe and the second detection probe form a double-antibody sandwich structure with the target protein in the sample to be detected. The double-antibody sandwich structure brings the pairing sequences of the single stranded DNA closer together and enable them to complementarily pair with each other, so as to form a stem-loop structure.

Some embodiments of the present disclosure also provide a kit for detecting a target protein, which includes a first container, a second container, a third container, a fourth container, and a fifth container.

The first container stores at least a conjugate of a first single stranded DNA and a first antibody.

The second container stores at least a conjugate of a second antibody, a second single stranded DNA, and an acceptor fluorescent molecule. The first antibody and the second antibody are capable of forming a double-antibody sandwich structure with the target protein under a condition that the target protein is present.

The third container stores at least a conjugate of a donor fluorescent molecule and a third single stranded DNA. The first single stranded DNA, the second single stranded DNA, and the third single stranded DNA are capable of forming a stem-loop structure under a condition that the double-antibody sandwich structure is formed. The spatial conformation of the stem-loop structure allows the donor fluorescent molecules and the acceptor fluorescent molecules to be located on the same side of the stem-loop structure, and the spatial distance between the two is less than the limit distance at which fluorescence resonance energy transfer can occur, for example, in the range of 70 angstroms to 99 angstroms.

The fourth container stores at least an antioxidant capable of inhibiting oxidation of the donor fluorescent molecule. Optionally, the antioxidant may be modified on the carrier molecules. Optionally, the carrier molecules may be selected from graphene oxides.

The fifth container stores at least an oxidant. The oxidant is capable of oxidizing the donor fluorescent molecule to emit a first fluorescence. The oxidants and the antioxidants are not present simultaneously in the sample to be detected. The antioxidants and carrier molecules need to be removed from the sample to be detected before adding the oxidants.

In the embodiments of the present disclosure, the detection principle of the kit for detecting a target protein is as follows:

Under a condition that the target protein is contained in the solution to be detected, the first antibody and the second antibody can form a double-antibody sandwich structure with the target protein. The first single stranded DNA, the second single stranded DNA, and the third single stranded DNA can form a stem-loop structure. The six pairing sequences in the three single stranded DNA are complementarily paired in pairs to form the stem of the stem-loop structure, and the remaining unpaired sequences form the loop of the stem-loop structure. The two paired sequences inside each single stranded DNA cannot be complementarily paired with each other and can only be paired with the pairing sequences of the other single stranded DNA. The spatial conformation of the stem-loop structure allows the donor fluorescent molecules and the acceptor fluorescent molecules are located on the same side of the stem-loop structure and the distance between the donor fluorescent molecules and the acceptor fluorescent molecules can ensure the fluorescence resonance energy transfer between the donor fluorescent molecules and the acceptor fluorescent molecules. Under the condition of removing the fourth detection reagent, the oxidant can oxidize the donor fluorescent molecules and cause it to emit a first fluorescence. The first fluorescence excites the acceptor fluorescent molecules to emit a second fluorescence based on a fluorescence resonance energy transfer effect, so that the content of the target protein can be obtained according to the intensity of the second fluorescence.

Under a condition that the target protein is not contained in the solution to be detected, the double-antibody sandwich structure and the stem-loop structure cannot be formed. Even if the antioxidant is removed and the oxidant is added to enable the donor fluorescent molecules to emit the first fluorescence, the distance between the donor fluorescent molecules and the acceptor fluorescent molecules is larger than the limit distance at which the fluorescence resonance energy transfer can occur, the donor fluorescent molecules cannot excite the acceptor fluorescent molecules to emit the second fluorescence. The failure to obtain the intensity of the second fluorescence indicates that the content of the target protein in the solution to be detected is zero, which further indicates that the solution to be detected does not contain the target protein.

In the embodiments of the present disclosure, the acceptor fluorescent molecules can only emit the second fluorescence under the excitation of the excitation light. Without the excitation of the first fluorescence, the acceptor fluorescent molecules will not emit the second fluorescence used to measure the content of the target protein. Therefore, the acceptor fluorescent molecules, as the detection fluorescent molecules, do not emit the background fluorescence that is easy to interfere with the measurement results, which ensures the reliability of using the second fluorescence as the detection fluorescence.

In some embodiments of the present disclosure, the donor fluorescent molecules may be acridinium esters and the acceptor fluorescent molecules may be quantum dots.

In some embodiments of the present disclosure, the acridinium ester has a maximum emission wavelength of 430 nm; and the quantum dot has a maximum absorption wavelength of 470 nm and a maximum emission wavelength of 605 nm.

In some embodiments of the present disclosure, the first single stranded DNA includes a first pairing sequence and a second pairing sequence. The first antibody is capable of specifically binding to a first epitope of a target protein. The second single stranded DNA has a third pairing sequence and a fourth pairing sequence, and the third pairing sequence is complementary to the second pairing sequence. The second antibody can specifically bind to the second epitope of the target protein. The third single stranded DNA includes a fifth pairing sequence and a sixth pairing sequence, the fifth pairing sequence is complementary to the fourth pairing sequence, and the sixth pairing sequence is complementary to the first pairing sequence. At least a part or all of the first pairing sequence, the second pairing sequence, the third pairing sequence, the fourth pairing sequence, the fifth pairing sequence, and the sixth pairing sequence includes bases ᵢₛₒG and ᵢₛₒC, ᵢₛₒG replaces the natural base G, and ᵢₛₒC replaces the natural base C, thereby preventing background fluorescence phenomenon caused by mismatches.

Some embodiments of the present disclosure provide a system for detecting a target protein, which includes: a reaction vessel, a microinjection pump, a filter, an optical signal detection module, and a calculation module.

The reaction vessel includes an accommodating chamber capable of accommodating a solution to be detected.

The microinjection pump is communicated with the accommodating chamber through an injection pipeline for injecting a mixture of a first detection probe, a second detection probe, a third detection probe, and a fourth detection probe into the accommodating chamber, so as to be mixed with the solution to be detected. The first detection probe is formed by coupling at least a first single stranded DNA and a first antibody. The second detection probe is formed by coupling at least a second antibody, a second single stranded DNA, and an acceptor fluorescent molecule in sequence. The third detection probe is formed by coupling at least a donor fluorescent molecule and a third single stranded DNA. The fourth detection probe includes an antioxidant for inhibiting the donor fluorescent molecule from emitting a first fluorescence.

The filter is disposed on an emergent light path of the first fluorescence, allowing a second fluorescence having a same wavelength as a maximum emission wavelength of the acceptor fluorescent molecule to pass through.

The optical signal detection module is disposed on an emergent light path of the first fluorescence and located on a downstream side of the filter to acquire the second fluorescence transmitted from the filter. The intensity of the second fluorescence may be zero, which means that the target protein is not present in the solution to be detected. Since the acceptor fluorescent molecules emit light under excitation, instead of emit light under oxidization. In the absence of excitation light, the acceptor fluorescent molecules themselves do not emit the second fluorescence, as a result, the background fluorescence noise will not be produced. Therefore, the intensity of the second fluorescence can reach zero in the absence of the target protein. In a case that the first fluorescence is used as the detection fluorescence, since the first fluorescence is produced under oxidization, and there are oxidizing substances in the solution to be detected, there will be background fluorescence noise in the first fluorescence, so that the intensity of the first fluorescence is generally not zero in the absence of the target protein. Therefore, using the second fluorescence as the detection fluorescence is more accurate and there is less error than using the first fluorescence as the detection fluorescence.

The calculation module converts the second fluorescence into a digital signal and obtains a content of the target protein in the solution to be detected based on a functional relationship between fluorescence intensity and protein content.

By adopting the above technical solutions, some embodiments of the present disclosure can achieve the following technical effects.

Some embodiments of the present disclosure adopt a combination of an immune response and a fluorescence resonance energy transfer effect to detect whether the solution to be detected contains a target protein. The details are as follows.

Under a condition that the target protein is contained in the solution to be detected, the first antibody, the second antibody, and the target protein form a double-antibody sandwich structure based on the immune reaction, so that the distance between the first single stranded DNA, the second single stranded DNA, and the third single stranded DNA is pulled closer, triggering DNA assembly and forming a stem-loop structure. The stem-loop structure allows the donor fluorescent molecules and the acceptor fluorescent molecules to be located on the same side of the stem-loop structure, thereby generating a fluorescence resonance energy transfer phenomenon between the two and causing the acceptor fluorescent molecules to emit the second fluorescence as the fluorescence signal to be measured. The content of the target protein in the solution to be detected is obtained according to the preset functional relationship between the fluorescence intensity and the protein content and the intensity of the fluorescence signal to be detected.

Under a condition that the target protein is not present in the solution to be detected, the double-antibody sandwich structure and the stem-loop structure cannot be formed, and the acceptor fluorescent molecules will not be excited by the excitation light to emit the second fluorescence, so there is no way to capture the fluorescent signal to be detected, indicating that the target protein is not present in the solution to be detected. In addition, even if the donor fluorescent groups emit background fluorescence under the action of certain oxidizing substances, since the stem-loop structure cannot be formed, the space distance between the donor fluorescent groups and the acceptor fluorescent groups is greater than the minimum distance required for the fluorescence resonance energy transfer effect, and the fluorescence resonance energy transfer effect will not occur between the two fluorescent groups, and the acceptor fluorescent groups will not emit the second fluorescence based on the background fluorescence. Therefore, qualitative detection of whether the target protein exists in the solution to be detected and quantitative detection of the content of the target protein in the solution to be detected are realized.

In various embodiments of the present disclosure, the second fluorescence emitted by the acceptor fluorescent molecules that are less likely to generate the background fluorescent noise is used as the fluorescent signal to be measured, and the first fluorescence emitted by the donor fluorescent molecules that usually have background fluorescent noise is not used as the fluorescent signal to be measured, so that the influence of the background fluorescent noise on the detection results can be greatly reduced, thus improving detection sensitivity and reducing detection errors, as a result, the measured protein content is closer to the real value.

### BRIEF DESCRIPTION OF THE DRAWINGS

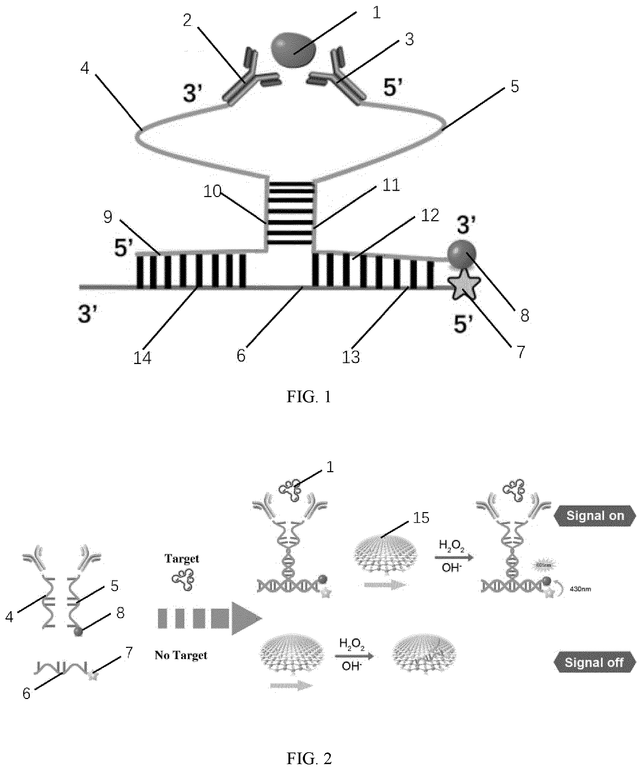

FIG. 1 is a schematic diagram of an immune sandwich structure and a stem-loop structure according to some embodiments of the present disclosure.

FIG. 2 is a schematic diagram of a detection method according to some embodiments of the present disclosure. In FIG. 2, in a case that the target protein is present (Target shown in FIG. 2), the antibody and the target protein form a double-antibody sandwich structure, and a stem-loop structure is formed between single stranded DNA molecules. After eluting the graphene oxide modified by the antioxidant, an oxidant (an alkaline solution of hydrogen peroxide) is added, and the acridinium ester emits a first fluorescence of 430 nm, thereby exciting quantum dots to emit the second fluorescence of 605 nm, which is referred to as a signal on state. In a case that the target protein is not present (No Target shown in FIG. 2), there will not preduce a double-antibody sandwich structure and a stem-loop structure. After eluting the graphene oxide modified by the antioxidant, the third single stranded DNA is adsorbed on the surface of the graphene oxide through π-π stacking, so that the third single stranded DNA will be eluted along with the graphene oxide. A conjugate of an acridinium ester and a third single stranded DNA is present in the eluted liquid, the acridinium ester will be oxidized by oxidizing substances in the liquid to produce background fluorescence, while a conjugate of a second antibody, a second single stranded DNA and a quantum dot will be present in the remaining liquid after elution. The quantum dots do not produce fluorescence due to the absence of excitation light, which is now referred to as a signal off state, indicating that there is no target protein in the liquid.

Reference numerals: a target protein 1, a first antibody 2, a second antibody 3, a first single stranded DNA 4, a second single stranded DNA 5, a third single stranded DNA 6, a donor fluorescent molecule 7, an acceptor fluorescent molecule 8, a first pairing sequence 9, a second pairing sequence 10, a third pairing sequence 11, a fourth pairing sequence 12, a fifth pairing sequence 13, a sixth pairing sequence 14, a complex of graphene oxide and an antioxidant, 15 (i.e., a fourth detection probe).

### DETAILED DESCRIPTION

The technology of each embodiment of the present disclosure will be described in detail below in combination with specific embodiments. It should be understood that the following specific embodiments are only used to help those skilled in the art to understand the present disclosure, rather than limiting the present disclosure. In addition, the following detailed embodiments may be arbitrarily combined without creative labor to form new embodiments.

### [Reagent composition for detecting a target protein]

Some embodiments of the present disclosure provide a reagent composition. The reagent composition is used to detect whether a target protein exists in the solution to be detected, thereby achieving qualitative detection of the target protein. Moreover, the concentration of the target protein in the solution to be detected can be obtained by using the reagent composition under the premise that the target protein exists in the solution to be detected, thereby realizing quantitative detection of the content of the target protein.

In some embodiments of the present disclosure, the solution to be detected may be from a blood sample or other non-blood sample. The blood sample may be from a whole blood sample, a serum sample, or a plasma sample.

In some embodiments of the present disclosure, the type of the target protein to be detected is not particularly limited. Illustratively, the target protein can be troponin, procalcitonin, or thyroid stimulating hormone, etc. In some embodiments of the present disclosure, the molecular weight of the target protein to be detected may range from 5 KD to 1500 KD, or range from 10 KD to 1200 KD, or range from 100 KD to 1000 KD, or range from 200 KD to 500 KD.

In some embodiments of the present disclosure, the reagent composition includes a first detection probe, a second detection probe, a third detection probe, and a fourth detection probe.

The first detection probe is formed by coupling at least a first single stranded DNA and a first antibody. The ribose ring at 3' end of the first single stranded DNA is modified by a NH2C7 modifying group, and is at least covalently linked to the amino group of the first antibody through a first coupling agent. The NH2C7 modifying group modifies the ribose ring at 3' end of the first single stranded DNA, but does not modify the base. The first coupling agent may be suberate bis(sulfosuccinimidyl) sodium salt. The amino group covalently linked to the first coupling agent in the first antibody is preferably the amino group of a heavy chain constant region, and of course it is not limited to the amino group of the heavy chain constant region, but may also be the amino group of a light chain. However, the coupled amino group is not the amino group of a complementarity determining region (CDR), otherwise it is difficult to bind to the epitope. The 3' end of the first single stranded DNA is not modified.

In some embodiments, the full-length sequence of the first single stranded DNA is shown in SEQ ID No: 1. The specific sequence may be ACGCTGAGTTATCAACGACTTTTTTTATCACATCAGGCTCTAGCGTATGCTATTG, but is not limited to the above sequences.

In some embodiments, the first single stranded DNA includes a first pairing sequence and a second pairing sequence. In the first single stranded DNA, the first pairing sequence is located upstream of the second pairing sequence in an orientation from the 5' end to 3' end. Illustratively, the first pairing sequence may be GCTGAGTT from the from 5' end to 3' end, and the second pairing sequence is CAACGAC from the from 5' end to 3' end. However, complementary pairing does not occur between the first pairing sequence and the second pairing sequence. In each embodiment of the present disclosure, complementary pairing does not occur between two pairing sequences contained within each single stranded DNA. Complementary pairing occurs only between single stranded DNAs in different detection probes, thereby assembling into a stem-loop structure.

In some embodiments, the first single stranded DNA has 55 nucleotides, the first pairing sequence covers the 3rd to 10th base sites of the first single stranded DNA starting from the 5' end, and the second pairing sequence covers the 13th to 19th base sites of the first single stranded DNA starting from the 5' end.

In some embodiments, the first antibody is capable of specifically binding to a first epitope of the target protein. The first antibody may be a monoclonal antibody capable of having a specific affinity for the first epitope of the target protein, which recognizes only the specific first epitope of the target protein, but does not recognize other epitopes of the target protein.

The second detection probe may be formed by coupling at least a second antibody, a second single stranded DNA, and an acceptor fluorescent molecule in sequence.

In some embodiments, the second antibody is capable of specifically binding to a second epitope of the target protein, and the first epitope is different from the second epitope. The second antibody may be a monoclonal antibody capable of having affinity for the second epitope of the target protein, which recognizes only a particular second epitope of the target protein, but does not recognize other epitopes of the target protein. Since the first antibody and the second antibody recognize different epitopes, respectively, when the target protein is present in the solution to be detected, the first antibody and the second antibody can react immunologically with the target protein to form a double-antibody sandwich structure.

In some embodiments, the full-length sequence of the second single stranded DNA is shown in SEQ ID No: 2. The specific sequence may be TACGTCCAGAACTTTACCAAACCACACCCTTTTTTTGTCGTTGGCTGAGATTC, but is not limited to the above sequences.

In some embodiments, the second single stranded DNA has a third pairing sequence and a fourth pairing sequence. In the second single stranded DNA, the third pairing sequence is located upstream of the fourth pairing sequence in an orientation from the 5' end to 3' end. Illustratively, the third pairing sequence is GTCGTTG from the 5' end to 3' end, and the fourth pairing sequence is GCTGAGAT from the 5' end to 3' end. The third pairing sequence is complementary to the second pairing sequence. There is no complementary relationship between the third pairing sequence and the fourth pairing sequence, thus reducing the possibility of mismatch within the DNA strand.

In some embodiments, the second single stranded DNA has 53 nucleotides. The third pairing sequence covers 37th to 43rd base sites starting from the 5' end of the second single stranded DNA, and the fourth pairing sequence covers 44th to 51th base sites starting from the 5' end of the second single stranded DNA.

In some embodiments, the 3' end of the second single stranded DNA is linked to an acceptor fluorescent molecule. Illustratively, the ribose ring at the 3' end of the second single stranded DNA is modified by a sulfhydryl group, a surface of the acceptor fluorescent molecule is modified by an amino group, and the sulfhydryl group is covalently linked to the amino group on the surface of the acceptor fluorescent molecule through a third coupling agent. The third coupling agent is 4-(N-maleimidomethyl)cyclohexane-1-carboxylic acid N-hydroxysuccinimide ester.

In some embodiments, the ribose ring at the 5' end of the second single stranded DNA is modified by a NH2C6 modifying group, and the NH2C6 modifying group is covalently linked to the amino group of the second antibody through a second coupling agent. The second coupling agent is suberate bis(sulfosuccinimidyl) sodium salt. The amino group covalently linked to the second coupling agent in the second antibody is preferably the amino group of the heavy chain constant region, and of course it is not limited to the amino group of the heavy chain constant region, but may also be the amino group of a light chain. However, the coupled amino group is not the amino group of the complementarity determining region (CDR), otherwise it is difficult to bind to the epitope.

The third detection probe may be formed by coupling at least a donor fluorescent molecule and a third single stranded DNA.

In some embodiments, the ribose ring at 5' end of the third single stranded DNA is modified by the NH2C6 modifying group, and the NH2C6 modifying group is covalently linked to the donor fluorescent molecule.

In some embodiments, the full-length sequence of the third single stranded DNA is shown in SEQ ID No: 3. The specific sequence may be CGATCTCAGCAACTCAGCAGCG, but is not limited to the above sequences.

In some embodiments, the third single stranded DNA includes a fifth pairing sequence and a sixth pairing sequence, and the fifth pairing sequence and the sixth pairing sequence are not complementary.

In some embodiments, in the third single stranded DNA, the fifth pairing sequence is located upstream of the sixth pairing sequence in an orientation from 5' end to 3' end.

In some embodiments, the third single stranded DNA has 22 nucleotides, the fifth pairing sequence covers the 3rd to 10th base sites of the third single stranded DNA starting from the 5' end, and the sixth pairing sequence covers the 11th to 18th base sites of the third single stranded DNA starting from the 5' end. The sequence of the fifth pairing sequence from the 5' end to 3' end may be ATCTCAGC. The sequence of the sixth pairing sequence from the 5' end to 3' end may be AACTCAGC.

In some embodiments, the fifth pairing sequence is complementary to the fourth pairing sequence, the sixth pairing sequence is complementary to the first pairing sequence, and the third pairing sequence is complementary to the second pairing sequence. In a case that a target protein is contained in the solution to be detected, the first antibody, the second antibody and the target protein form a double-antibody sandwich structure. The formation of the double-antibody sandwich structure and DNA assembly among the three single stranded DNAs, enables the formation of the stem-loop structure between the three single stranded DNAs with the help of the complementary pairing relationship between the above-mentioned DNA sequences. As a result, the donor fluorescent molecules and the acceptor fluorescent molecules are located on the same side of the stem-loop structure, and fluorescence resonance energy transfer (FRET) occurs between the two types of fluorescent molecules.

In some embodiments, the donor fluorescent molecules emit a first fluorescence under a condition that it can be oxidized by an oxidant, and the first fluorescence excites the acceptor fluorescent molecules to emit a second fluorescence according to a fluorescence resonance energy transfer effect under a condition that the first single stranded DNA, the second single stranded DNA, and the third single stranded DNA are paired with each other, so as to obtain the content of the target protein according to the intensity of the second fluorescence. In a case that the target protein is not present in the solution to be detected, even if the donor fluorescent molecules hane a strong background fluorescence, the distance between the donor fluorescent molecules and the acceptor fluorescent molecules does not meet the conditions for the generation of fluorescence resonance energy transfer, and therefore, the acceptor fluorescent molecules will not produce the second fluorescence. In a case that the intensity of the second fluorescence is not obtained from the solution to be detected, it means that the target protein is not present in the solution to be detected, thereby avoiding the false positive caused by the background fluorescence when only one type of fluorescent groups is used for detection. In a case that the target protein is present in the solution to be detected, two antibodies form a stable double-antibody sandwich structure with the target protein (as an antigen), and three single stranded DNAs form a stem-loop structure, the two structures enables the distance between the donor fluorescent molecules and the acceptor fluorescent molecules to be sufficient to produce a fluorescence resonance energy transfer effect, thereby determining the content of the target protein based on the intensity of the second fluorescence emitted by the acceptor fluorescent molecules and a standard curve between the intensity and content of the target protein.

In some embodiments, the donor fluorescent molecules emit fluorescence without the irradiation of excitation light, but emit light under oxidation of the oxidant. However, the fluorescence emitted by the acceptor fluorescent molecules needs the irradiation of excitation light. Without the irradiation of excitation light, the acceptor fluorescent molecules themselves will not actively emit fluorescence, so it is not easy to generate background fluorescence. Therefore, the measurement results are not interfered by the background fluorescence of the acceptor fluorescent molecules. Compared with a method in which fluorescence is emitted by only one type of fluorescent group, the methods of various embodiments of the present disclosure can effectively avoid the interference of the background fluorescence of the fluorescent molecules, so the measurement results are more accurate. The excitation light of the acceptor fluorescent molecules comes from the first fluorescence emitted by the donor fluorescent molecules, but the first fluorescence alone is not sufficient to enable the acceptor fluorescent molecules to emit the second fluorescence. Even if the donor fluorescent molecules have background fluorescence, if the distance between the donor fluorescent molecules and the acceptor fluorescent molecules is greater than the minimum distance required for the fluorescence resonance energy transfer effect, the background fluorescence will not excite the donor fluorescent molecules to emit the second fluorescence. However, in a case that the target protein is present in the solution to be detected, the stem-loop structure enables the distance between the donor fluorescent molecules and the acceptor fluorescent molecules to be smaller than the minimum distance required for the fluorescence resonance energy transfer effect, and then the donor fluorescent molecules can excite the acceptor fluorescent molecules to emit the second fluorescence. In summary, various embodiments of the present disclosure can effectively avoid the influence of background fluorescence of the donor fluorescent molecules on the detection result, thereby reducing the measurement error.

In some embodiments, during detection, the donor fluorescent molecules and the acceptor fluorescent molecules need to be able to undergo fluorescence resonance energy transfer, and the first detection probe, the second detection probe, and the third detection probe need to be complementarily paired in the presence of the target protein to form a stem-loop structure, so that the distance between the donor fluorescent molecules and the acceptor fluorescent molecules is less than or equal to 100 angstroms. At this time, the first fluorescence emitted by the donor fluorescent molecules can become the excitation light of the acceptor fluorescent molecules, thereby exciting the acceptor fluorescent molecule to produce the second fluorescence (as shown in FIG. 1).

In some embodiments, the donor fluorescent molecules may be acridinium esters. The acridinium ester emits fluorescence without the need for excitation light, but achieves emiting light under oxidization in the presence of an oxidant. Since there may be oxidizing substances in the solution to be detected, acridinium ester can also be oxidized and emit light, so acridinium ester will have background fluorescence. If the fluorescence of the acridinium ester is used as the measured fluorescence, the background fluorescence will interfere with the measured fluorescence. Therefore, in order to further improve the sensitivity of the detection and reduce the measurement error, the fluorescence of the acridinium ester is not used as the measured fluorescence in the embodiments of the present disclosure. The chemiluminescent substrate of the acridinium ester is an alkaline solution of H₂O₂, also known as an oxidant. In a case that the acridinium ester coexists with the alkaline solution of H₂O₂, the molecules of the acridinium ester are attacked by hydrogen peroxide ions, the acridinium ester can react with hydrogen peroxide (H₂O₂) to form unstable dioxyethane, which subsequently decomposes to emit fluorescent light.

In some embodiments, the maximum emission wavelength of the donor fluorescent molecules, i.e. acridinium esters is 430 nm. In some embodiments, the acceptor fluorescent molecules may be quantum dots. The maximum absorption wavelength of the acceptor fluorescent molecules may range from 420 nm to 520 nm, for example, it can be 470 nm. The maximum emission wavelength of the acceptor fluorescent molecules may range from 595 nm to 615 nm, for example, it can be 605 nm. Therefore, the donor fluorescent molecules and the acceptor fluorescent molecules in the embodiments of the present disclosure can undergo a fluorescence resonance energy transfer effect. In some embodiments, the quantum dots are core-shell quantum dots having a core layer material selected from one or more of CdSe, CdS, CdTe, CdSeTe, CdZnS, ZnTe, CdSeS, PbS, and PbTe, and a shell layer material selected from one or more of ZnS, ZnSe, ZnSeS, PbS, and PbSeS. In some embodiments, the particle sizes of the quantum dots may range from 3 to 5 nm, illustratively, may be 4.1 nm.

In some embodiments, G in the first single stranded DNA, the second single stranded DNA, and the third single stranded DNA may be replaced by ᵢₛₒG, and C in the first single stranded DNA, the second single stranded DNA, and the third single stranded DNA may be replaced by ᵢₛₒC. Since in a case that the solution to be detected is from a blood sample or the like, and natural nucleic acids are also present in the sample, the introduction of non-natural base pairs can avoid non-specific binding of the first single stranded DNA, the second single stranded DNA, and the third single stranded DNA with the nucleic acids in the blood sample, thereby avoiding measurement errors caused by DNA mismatch. In addition, these non-natural base pairs do not affect the pairing between the first single stranded DNA, the second single stranded DNA, and the third single stranded DNA, and can still form a stable stem-loop structure, thereby not affecting the production of the second fluorescence. Illustratively, G in the full-length sequence of the first single stranded DNA is replaced by ᵢₛₒG, and C is replaced by ᵢₛₒC. G in the full-length sequence of the second single stranded DNA is replaced by ᵢₛₒG, and C is replaced by ᵢₛₒC. G in the full-length sequence of the third single stranded DNA is replaced by ᵢₛₒG, and C is replaced by ᵢₛₒC.

In some embodiments, G in the first pairing sequence and the sixth pairing sequence that are paired with each other is replaced by ᵢₛₒG, and C is replaced by ᵢₛₒC. Illustratively, the first pairing sequence is ᵢₛₒGᵢₛₒCTᵢₛₒGAᵢₛₒGTT from the 5' end to 3' end, and the sixth pairing sequence is AAᵢₛₒCTᵢₛₒCAᵢₛₒGᵢₛₒC from the 5' end to 3' end.

In some embodiments, G in the second pairing sequence and the third pairing sequence that are paired with each other is replaced by ᵢₛₒG, and C is replaced by ᵢₛₒC. Illustratively, the second pairing sequence is ᵢₛₒCAAᵢₛₒCᵢₛₒGAᵢₛₒC from the 5' end to 3' end, and the third pairing sequence is ᵢₛₒGTᵢₛₒCᵢₛₒGTTᵢₛₒG from the 5' end to 3' end.

In some embodiments, G in the fourth pairing sequence and the fifth pairing sequence that are paired with each other is replaced by ᵢₛₒG, and C is replaced by ᵢₛₒC. Illustratively, the fourth pairing sequence is ᵢₛₒGᵢₛₒCTᵢₛₒGAᵢₛₒGAT from the 5' end to 3' end, and the fifth pairing sequence is ATᵢₛₒCTᵢₛₒCAᵢₛₒGᵢₛₒC from the 5' end to 3' end.

In the above embodiments, ᵢₛₒG has a structural formula as follows: 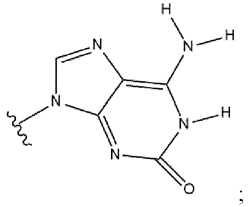 and the polyline indicates an attachment site.

ᵢₛₒC has a structural formula as follows:

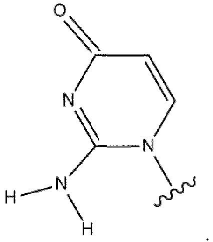

A bonding form of ᵢₛₒG and ᵢₛₒC is as follows: 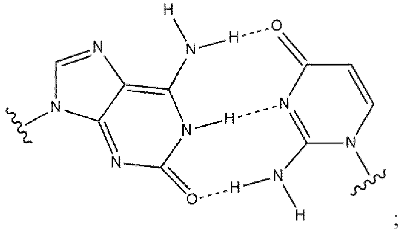 wherein 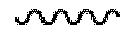 indicates a site connecting to deoxyribose in a DNA molecule.

Therefore, complementary pairing can be generated between ᵢₛₒG and ᵢₛₒC, which does not affect the mutual pairing between two of the first single stranded DNA, the second single stranded DNA, and the third single stranded DNA, but can prevent mismatch between each of the first single stranded DNA, the second single stranded DNA, and the third single stranded DNA and the natural nucleic acid in the solution to be detected. Therefore, in the above-described embodiments, measurement errors caused by mismatch between the single stranded DNA and the natural nucleic acid molecule can be reduced.

The fourth detection probe includes an antioxidant for inhibiting the donor fluorescent molecule from emitting the first fluorescence. During detection, it is necessary to add a first detection probe, a second detection probe, a third detection probe, and a fourth detection probe into the solution to be detected, and mix to form a sample to be detected. In a case that an antioxidant is present in the sample to be detected, even if the first detection probe, the second detection probe, and the third detection probe form a stem-loop structure, the donor fluorescent molecules, i.e. acridinium esters will not emit fluorescence because the antioxidant will inhibit oxidation emission of the acridinium esters. Therefore, it is necessary to remove the antioxidant from the sample to be detected before detection.

In some embodiments, in order to smoothly remove the antioxidant from the sample to be detected, the fourth detection probe further includes a carrier molecule, and the antioxidant is bound to the surface of the carrier molecule. At this time, since the carrier molecule has a large molecular weight, it is relatively easy to remove from the sample to be detected. As long as the carrier molecules are removed, the antioxidant can be removed at the same time.

In some embodiments, the carrier molecule may be graphene oxide (GO). The carboxyl group on the graphene oxide is bonded to the hydroxyl group on the antioxidant through a sulfoxide condensing agent, and the carboxyl group on the graphene oxide is bonded to an amino group on the antioxidant through 1-(3-dimethylaminopropyl)-3-ethylcarbodiimide hydrochloride. After the first detection probe, the second detection probe, the third detection probe, and the fourth detection probe are added to the solution to be detected, a sample to be detected is formed. The third single stranded DNA is adsorbed on the surface of the graphene oxide through π-π stacking, and the acridinium ester (AE) labeled at its end cannot be oxidized to emit light due to the presence of the antioxidants. Even if a small part of the acridinium esters produces chemiluminescence, that is, background fluorescence with a wavelength of 430 nm, it will not affect the measurement results. Since the fluorescence obtained during detection comes from the acceptor fluorescent molecular quantum dots, but not from the acridinium esters, only the fluorescence emitted by the quantum dots can be obtained by setting a filter, while the background fluorescence of acridinium ester can be filtered out. In addition, even if a small part of the acridinium ester has background fluorescence, the acridinium ester will not excite the quantum dots to emit fluorescence based on fluorescence resonance energy transfer without the presence of the stem-loop structure. Thus, in the embodiments of the present disclosure, the influence of background fluorescence of the acridinium ester on the measurement result can be greatly reduced, thereby reducing the measurement errors. In some embodiments, the fluorescence emitted by the quantum dots may be obtained by a filter and a photomultiplier tube (PMT). Illustratively, if the fluorescence emitted by the quantum dots is 605 nm, the filter only allows light with a wavelength of 605 nm to pass through.

In some embodiments, the antioxidant is selected from any one or more of cannabidiol, vitamin C, vitamin E, tea polyphenol and glutathione. The oxidant includes an alkaline solution of hydrogen peroxide. After removing the antioxidant, the added oxidant can enable the donor fluorescent molecules, i.e. acridine esters to emit fluorescence.

According to the embodiments of the present disclosure, the antibody recognizes the target protein through an immune response, so that the distance between a pair of DNAs coupled to the antibody is shortened, thereby initiating DNA assembly, triggering cascade DNA assembly, and causing a fluorescence resonance energy transfer effect between two fluorescent molecules to produce a detection signal.

In each embodiment of the present disclosure, acridinium esters (AE) are used as fluorescent energy donors, and fluorescent quantum dots (QDs) are used as fluorescent energy acceptors according to a chemiluminescence resonance energy transfer system. Amplification of the fluorescent signal is achieved through immune response and DNA self-assembly, and detection of the content of the target protein is converted to detection of the intensity of the fluorescent signal. The methods in the embodiments of the present disclosure may be carried out homogeneously. Compared with the conventional fluorescence resonance energy transfer analysis method, the detection method in the embodiments of the present disclosure is simple and rapid, and does not require expensive laser light. In the embodiments of the present disclosure, the second fluorescence is used as the detection fluorescence, which reduces the background interference of the first fluorescence. In the embodiments of the present disclosure, an immune response is used to enrich the target protein, which has good selectivity for the target protein and high detection sensitivity.

### [Method for detecting a target protein]

As shown in FIG. 2, some embodiments of the present disclosure provide a method for detecting a target protein, which can use the above-mentioned reagent compositions. Features of the above-mentioned reagent compositions may be incorporated in this section. The detection method in the above embodiments includes the steps as follows:
(1) Providing a first detection probe, a second detection probe, a third detection probe, and a fourth detection probe. The first detection probe is formed by coupling at least a first single stranded DNA and a first antibody. The second detection probe is formed by coupling at least a second antibody, a second single stranded DNA, and an acceptor fluorescent molecule in sequence. The third detection probe is formed by coupling at least a donor fluorescent molecule and a third single stranded DNA.
(2) Adding a first detection probe, a second detection probe, a third detection probe and a fourth detection probe into the solution to be detected, and mixing to form a sample to be detected. Under a condition that the target protein is contained in the solution to be detected, the first antibody, the target protein and the second antibody form a double-antibody sandwich structure; the first single stranded DNA, the second single stranded DNA and the third single stranded DNA form a stem-loop structure, so that the donor fluorescent molecules and the acceptor fluorescent molecules are located on the same side of the stem-loop structure.
(3) Removing the fourth detection probe from the sample to be detected, adding an oxidant for oxidizing the donor fluorescent molecules to emit the first fluorescence, and collecting the second fluorescence according to the maximum emission wavelength of the acceptor fluorescent molecules. The fourth detection probe contains an antioxidant for inhibiting oxidation of the donor fluorescent molecules from being oxidized by the oxidizing substances in the solution to be detected. Since the donor fluorescent molecules do not emit the first fluorescence in the presence of the antioxidants, it is necessary to remove the antioxidants in advance before adding the oxidants. In addition, the donor fluorescent molecules will be protected by antioxidants before being oxidized, thereby reducing the background fluorescence emitted by the donor fluorescent molecules due to the influence of other factors.
(4) Determining that the target protein is not contained in the solution to be detected in a case that the second fluorescence is not collected; and obtaining a content of the target protein in the solution to be detected according to an intensity of the second fluorescence based on a functional relationship between fluorescence intensity of and protein content in a case that the second fluorescence is collected.

In this step, the donor fluorescent molecules will produce the first fluorescence after being oxidized. In order to avoid the influence of the first fluorescence on the detection result, a filter is used to filter out the first fluorescence, and the filter only allows the transmission of the second fluorescence emitted by the acceptor fluorescent molecules. At this time, the content of the target protein is obtained according to the intensity of the second fluorescence. If the relative intensity of the second fluorescence is zero, it means that the target protein is not contained in the solution to be detected. If the relative intensity of the second fluorescence is not zero, the content of the target protein in the solution to be detected is obtained according to the functional relationship between the relative intensity of the second fluorescence and the protein content. The relative intensity refers to the value of the remaining fluorescence intensity after the background fluorescence intensity of the control group is excluded from the intensity of the second fluorescence signal.

In some embodiments, the working concentration of the first detection probe in the sample to be detected may be 1 nM to 20 nM. The working concentration refers to the concentration of each detection probe during actual detection, i.e., the final concentration of each detection probe in the sample to be detected. The working concentration is not equal to the storage concentration of each detection probe in the kit for detecting a target protein. In some embodiments, the working concentration of the second detection probe in the sample to be detected is 1 nM to 20 nM. In some embodiments, the working concentration of the third detection probe in the sample to be detected is 0.05 nM to 0.2 nM. In some embodiments, the working concentration of the the working concentration of the second detection probe in the sample to be detected may be detection probe in the sample to be detected is 15 µg/ml to 25 µg/ml.

In some embodiments, the blood sample solution to be detected is from a whole blood sample, a serum sample, or a plasma sample.

In some embodiments, the time for mixing is from 5 minutes to 10 minutes. If the time for mixing is too short, the double-antibody sandwich structure and the stem-loop structure may not be completely formed, and the fluorescence resonance energy transfer between the donor fluorescent molecules and the acceptor fluorescent molecules cannot completely occur later, which will make the measured fluorescence value less than the actual value. If the time for mixing is too long, the double-antibody sandwich structure and the stem-loop structure may be damaged, which will also make the measured fluorescence value to be less than the actual value.

In some embodiments, the temperature for mixing may be from 36 °C to 37 °C.

In some embodiments, the volume of the oxidant may be 200 µL and the oxidant is an alkaline hydrogen peroxide solution with a pH of 8.0. The alkaline hydrogen peroxide solution is obtained by dissolving hydrogen peroxide in TBS buffer, wherein the final concentration of hydrogen peroxide is 0.1 M, and the final concentration of TBS is 10 mM.

In some embodiments, the target protein includes troponin, procalcitonin, or thyroid stimulating hormone. However, in some other embodiments, the target protein is not limited to the above mentioned ones. As long as the first antibody and the second antibody in the present disclosure can specifically bind to different epitopes of the target protein, respectively, and form a stable double-antibody sandwich structure. If the first antibody and the second antibody bind to two target proteins, respectively, the binding state is unstable, the binding is easy to break and re-bind. Finally, the first antibody and the second antibody only bind to one target protein at the same time, and a relatively stable double-antibody sandwich structure will be formed at this time. The double-antibody sandwich structure will in turn pull the first single stranded DNA, the second single stranded DNA, and the third single stranded DNA to form a stem-loop structure, and the donor fluorescent molecules and the acceptor fluorescent molecules are located on the same side of the stem-loop structure, so that the donor fluorescent molecules can excite the acceptor fluorescent molecules to produce the second fluorescence. Therefore, one target protein corresponds to one acceptor fluorescent molecule, and the number of the target proteins is linearly related to the fluorescence intensity of the acceptor fluorescent molecules, and the content of the target proteins can be calculated according to the linear equation contained in the standard curve (i.e., the functional relationship between the intensity of the second fluorescence and the protein content) and the fluorescence intensity of the obtained acceptor fluorescent molecules.

In some embodiments, specific detection steps are as follows: mixing the detection probe with a blood sample or a whole blood sample or a serum sample or a plasma sample containing the target proteins to be detected, incubating on a chemiluminescence detector at 37°C for 5 to 10 minutes, adding a chemiluminescent substrate hydrogen peroxide and sodium hydroxide (i.e., the above-mentioned oxidant), and collecting the generated chemiluminescence fluorescence signal through a PMT detection module in the chemiluminescence detector. The chemiluminescence detector automatically invokes the standard curve and reports the concentration of the target protein in the sample to be detected according to the functional relationship between the fluorescence intensity and the protein content contained in the standard curve.

### [Kit for detecting a target protein]

Some embodiments of the present disclosure provide a kit for detecting a target protein, which uses the detection method described above for detection. The kit for detecting a target protein includes a first container, a second container, a third container, a fourth container, a fourth container, and a fifth container.

The first container stores at least a conjugate of a first single stranded DNA and a first antibody. The first single stranded DNA contains 55 bases, and the 3' end is modified with a NH2C7 group. The 3'C7 amino modified primers are purchased from GenScript Biotech Co., Ltd. The NH2C7 group is covalently linked to the amino group of the first antibody through a first coupling agent, i.e., suberate bis(sulfosuccinimidyl) sodium salt (BS3), thereby forming a conjugate of the first single stranded DNA and the first antibody.

The second container stores at least a conjugate of a second antibody, a second single stranded DNA, and an acceptor fluorescent molecule. The first antibody and the second antibody are capable of forming a double-antibody sandwich structure with the target protein under a condition that the target protein is present. The second single stranded DNA contains 53 bases, with a sulfhydryl group modified at the 3' end (purchased from GenScript), and a NH2C6 group modified at the 5' end. The sulfhydryl-modified reagent 3' SH C6, 5'Aminolinker(C6) modified primers are purchased from GenScript Biotech Co., Ltd. The 5'-end modification is added to the 5' ribose ring in the form of an ammonium phosphite in the last step of the synthesis cycle via β-cyanoethyl chemical reaction, rather than to the last base. The sulfhydryl group at the 3' end is covalently linked to the amino group on the surface of the amino-modified quantum dots (QDs) via the third coupling agent, i.e. 4-(N-maleimidomethyl)cyclohexane-1-carboxylic acid N-hydroxysuccinimide ester.

In some embodiments, the model of the amino-modified water-soluble quantum dots may be amino-water-soluble quantum dots (PEG)-605, and the component is CdSe/ZnS, which is purchased from Xi'an Qiyue Biotechnology Co., Ltd. PEG)-605 has a maximum absorption peak wavelength of 470 nm, a maximum emission wavelength of 605 nm, a particle size of 4.1 nm, a absorption spectrum range of 470 nm±50 nm, and a emission spectrum range of 605 nm±10 nm. The amino group of the amino-modified quantum dot is coupled to the sulfhydryl group at the 3' end of the second single stranded DNA to form a conjugate of the second single stranded DNA and the quantum dots. The NH2C6 group at the 5' end of the second single stranded DNA is covalently linked to the amino group on the second antibody through the second coupling agent BS3 to form a conjugate of the second antibody, the second single stranded DNA, and the acceptor fluorescent molecules.

The third container stores at least a conjugate of a donor fluorescent molecule and a third single stranded DNA. The first single stranded DNA, the second single stranded DNA, and the third single stranded DNA are capable of forming a stem-loop structure under a condition that the double-antibody sandwich structure is formed. The donor fluorescent molecules and the acceptor fluorescent molecules can be located on the same side of the stem-loop structure. The third single stranded DNA contains 22 bases, with NH2C6 group modified at the 5' end. The modified primer 5'Aminolinker(C6) is added to the 5' ribose ring of the third single stranded DNA in the form of an ammonium phosphite in the last step of the synthesis cycle via β-cyanoethyl chemical reaction, rather than to the last base. The modified primers are purchased from purchased from GenScript Biotech Co., Ltd. A conjugate of a donor fluorescent molecule and the third single stranded DNA is formed by adding acridinium ester (NSP-DMAE-NHS). The donor fluorescent molecule is an acridinium ester, which is purchased from Suzhou Yaco Technology Co., Ltd., with CAS No. 194357-64-7. The 3rd to 10th base sites of the third single stranded DNA starting from the 5' end are completely complementary to the bases of the 3rd to 10th base sites of the second single stranded DNA starting from the 3' end (i.e., the 44th to 51th base sites starting from the 5' end). The 5th to 12th base sites of the third single stranded DNA starting from the 3' end (i.e., the 11th to 18th base sites starting from the 5' end) are completely complementary to the bases of the 3rd to 10th base sites of the first single stranded DNA starting from the 5' end, as shown in FIG. 1.

The fourth container stores at least an antioxidant capable of inhibiting oxidation of the donor fluorescent molecule. The antioxidant may be bound to the surface of the carrier molecule. In some embodiments, the carrier molecule may be graphene oxide.

The fifth container stores at least an oxidant capable of oxidizing the donor fluorescent molecule to emit a first fluorescence.

The detection principle of the kit for detecting a target protein in the above embodiments is as follows:

The third single stranded DNA has 8 bases complementary to the first single stranded DNA and the second single stranded DNA, respectively, wherein the natural base pairs G and C are replaced by the non-natural base pairs ᵢₛₒG and ᵢₛₒC.

In the absence of the target protein, a stem-loop structure will not be formed. The third single stranded DNA is adsorbed on the surface of the oxygenated graphene (GO) through π-π stacking, and the acridinium ester (AE) labelled at its end cannot be oxidized to emit light due to the presence of antioxidants, even a small part of the acridinium ester produces background fluorescence, and the chemiluminescence (with a wavelength of 430 nm) of the acridinium ester cannot be detected by the PMT due to the filter of the apparatus (only 605 nm light is allowed to pass through).

In the presence of the target protein, the first antibody and the second antibody can form a double-antibody sandwich structure with the target protein. Since the first antibody is coupled to the first single stranded DNA, the second antibody is coupled to the second single stranded DNA, the double-antibody sandwich structure enables the first single stranded DNA and the second single stranded DNA to be close enough to form an ortho-complex, and can hybridize with the third single stranded DNA, so that the first single stranded DNA, the second single stranded DNA, and the third single stranded DNA form a neck-loop structure, and the donor fluorescent molecules and the acceptor fluorescent molecules are located on the same side of the neck-loop structure. The complex formed by the antibody and DNA is not substantially adsorbed by the carrier molecule, i.e. graphene oxide, so the antioxidant coupled to the carrier molecule also does not substantially affect the luminescence of the acridinium ester. Under the condition of removing the fourth detection reagent, the added oxidant can oxidize the donor fluorescent molecules and cause it to emit a first fluorescence. The first fluorescence excites the acceptor fluorescent molecules to emit a second fluorescence (e.g., 605 nm) according to the fluorescence resonance energy transfer, so as to obtain the content of the target protein according to the intensity of the second fluorescence.

### [System for detecting a target protein]

The present disclosure provides a system for detecting a target protein, which includes a reaction vessel, a microinjection pump, a filter, and a calculation module.

The reaction vessel has an accommodating chamber capable of accommodating a solution to be detected.

The microinjection pump is communicated with the accommodating chamber through an injection pipeline for injecting a mixture of a first detection probe, a second detection probe, a third detection probe, and a fourth detection probe into the accommodating chamber. The first detection probe is formed by coupling at least a first single stranded DNA and a first antibody. The second detection probe is formed by coupling at least a second antibody, a second single stranded DNA, and an acceptor fluorescent molecule in sequence. The third detection probe is formed by coupling at least a donor fluorescent molecule and a third single stranded DNA. The fourth detection probe includes an antioxidant for inhibiting the donor fluorescent molecule from emitting a first fluorescence.

The filter is disposed on an emergent light path of the first fluorescence, allowing a second fluorescence having a same wavelength as a maximum emission wavelength of the acceptor fluorescent molecule to pass through.

The optical signal detection module is disposed on an emergent light path of the first fluorescence and located on a downstream side of the filter to acquire the second fluorescence transmitted from the filter.

The calculation module converts the second fluorescence into a digital signal and obtains a content of the target protein in the solution to be detected based on a functional relationship between fluorescence intensity and protein content.

The embodiments of the present disclosure provide a simple, rapid and sensitive homogeneous chemiluminescence immunoassay protein detection method through a dual quenching mechanism of graphene oxide coupled antioxidants and filters in combination with chemiluminescence resonance energy transfer and immunoassay techniques.

Some embodiments of the present disclosure have the following characteristics compared with existing immunoassay methods.
(1) Some examples of the present disclosure are homogeneous immunoassay methods, which are simple to operate and greatly shorten the turnaround time (TAT) of clinical tests samples. The whole blood can be loaded without centrifugation of the blood sample, and the detection report can be obtained in about 5 minutes.
(2) The acridinium esters in some embodiments of the present disclosure emit light by oxidation, and the quantum dots emit light by excitation, so that self-excitation luminescence between the acridinium esters and the quantum dots can be realized without the need for a complex external excitation light system, which can effectively reduce the complexity and cost of a measurement instrument. Since the requirements for supporting the auxiliary detection equipment are reduced, the number of modules is reduced, the cost is reduced, and the failure rate is greatly reduced, automatic detection or miniaturized portable bedside detection (POCT) can be realized.
(3) The acridinium ester-quantum dot luminescence system in some embodiments of the present disclosure introduces a dual quenching mechanism (graphene oxide-reducing agent and filter), which has lower background fluorescence and higher detection sensitivity, and is suitable for detection with higher sensitivity.
(4) In some embodiments of the present disclosure, the switching efficiency reaches 100% by introducing switching of the graphene oxide-antioxidant quenching mechanism through an immune response and introducing an external filter, combined with the chemiluminescence resonance energy transfer effect, so that quantum dots emit light without separation and cleaning steps.
(5) DNA molecules in some embodiments of the present disclosure contain non-natural base pairs (ᵢₛₒG and ᵢₛₒC) to avoid non-specific binding to the nucleic acid in the sample.

The technology of the present disclosure will be further illustrated below in combination with various preparation examples and embodimentss.

Preparation Example 1 Preparation of a conjugates of second single stranded DNA and acceptor fluorescent molecules (quantum dots)

This preparation example provides a method for preparing a conjugate of a second single stranded DNA and quantum dots, which includes the steps as follows:
1. Preparation of SMCC solution: 10 mg of 4-(N-maleimidomethyl)cyclohexane-1-carboxylic acid N-hydroxysuccinimide ester (SMCC) is weighed and dissolved in 1 mL DMF. SMCC is purchased from Shanghai Aladdin Biochemical Technology Co., Ltd., with a product number N159712 and CAS No. 64987-85-5.
2. Preparation of a second single stranded DNA solution: 10 µM of the second single stranded DNA is taken, and 1 mL of purified water is added to dissolve.
3. Preparation of the conjugate of a second single stranded DNA and quantum dots: 100 µL of the second single stranded DNA solution is taken and placed in an EP tube, 200 µL of QDs (purchased from Xi'an Qiyue Biotechnology Co., Ltd., amino-water-soluble quantum dots with a model number of (PEG)-605, with a concentration of 8 µM) is added therein, then 3 µL of SMCC solutionn is added therein, which are mixed homogeneously, and incubated at 37 °C for 30 min.
4. Dialysis: the coupled conjugate of the second single stranded DNA and quantum dots is sucked out from the EP tube and added to a dialysis bag (with a specification of 5 kd), which is tied and placed into a beaker containing 2 to 3 L TE solution (10 mM Tris, 1 mM EDTA, PH=8.0) for dialysis. The dialysis bag is soaked in advance. The dialysate is changed every 2 to 3 hours, and dialysis is performed for three times. After the dialysis is completed, the liquid in the dialysis bag is collected into a centrifuge tube and stored at 2 to 8 °C for later use.

Preparation Example 2 Preparation of a conjugate of the second donor fluorescent molecule (acridinium ester) and the third single stranded DNA

This preparation example provides a method for preparing a conjugate of an acridinium ester (AE) and a third single stranded DNA, which includes the steps as follows:
1. Preparation of a third single stranded DNA solution: 20 µM of the third single stranded DNA is taken, and 1 mL of purified water is added to dissolve.
2. Preparation of NHS-AE solution: 4 mg of acridinium ester (NSP-DMAE-NHS) is weighed and dissolved in 1 mL of purified water. The acridinium esters are purchased from Suzhou Yaco Technology Co., Ltd., with CAS No. 194357-64-7.
3. Coupling: 10 µL of the third single stranded DNA solution is added to every 1 mg NHS-AE solution, mixed homogeneously in EP tubes, and incubated at 37 °C for 30 min.
4. Dialysis: the coupled product is sucked out from the EP tube and added to a dialysis bag (with a specification of 5 kd), which is tied and placed into a beaker containing 2 to 3 L TE solution (10 mM Tris, 1 mM EDTA, PH=8.0) for dialysis (the dialysis bag is soaked in advance). The dialysate is changed every 2 to 3 hours, and dialysis is performed for three times. After the dialysis is completed, the liquid in the dialysis bag is collected into a centrifuge tube and stored at 2 to 8°C for later use.

Preparation Example 3 Preparation of a conjugate of the first single stranded DNA and the first antibody

This preparation example provides a method for preparing a conjugate of a first single stranded DNA and a first antibody, which includes the steps as follows:
1. Preparation of BS3 solution: 10 mg of suberate bis(sulfosuccinimidyl) sodium salt (BS3) is weighed into 1 mL of purified water. BS3 is purchased from Shanghai Aladdin Biochemical Technology Co., Ltd., with a product number of S304724.
2. Activattion of antibody: the packaged first antibody is removed, thawed and mixed through centrifugation. 3 µL of BS3 solution is added to every 1 mg of the first antibody, 6.5 µL of the first single stranded DNA is added therein and mixed homogeneously in the EP tube, and incubated at 37 °C for 30 min.
3. Dialysis: the conjugate of the first single stranded DNA and the first antibody is sucked out from the EP tube and added to a dialysis bag (with a specification of 100 kd), which is tied and placed into a beaker containing 2 to 3 L TE solution for dialysis (the dialysis bag is soaked in advance). The dialysate is changed every 2 to 3 hours, and dialysis is performed for three times. After the dialysis is completed, the liquid in the dialysis bag is collected into a centrifuge tube and stored at 2 to 8°C for later use.

Preparation Example 4 Preparation of a conjugate of the second antibody, the second single stranded DNA and acceptor fluorescent molecules

This preparation example provides a method for preparing a conjugate of a second antibody, a second single stranded DNA, and an acceptor fluorescent molecule, which includes the steps as follows:
1. Preparation of BS3 solution: 10 mg of suberate bis(sulfosuccinimidyl) sodium salt (BS3) is weighed into 1 mL of purified water. BS3 is purchased from Shanghai Aladdin Biochemical Technology Co., Ltd.
2. Activattion of antibody: the packaged second antibody is removed, thawed and mixed through centrifugation. 3 µL of BS3 solution is added to every 1 mg of second antibody, 6.5 µL of a conjugate of second single stranded DNA and quantum dots is added therein and mixed homogeneously in the EP tube, and incubated at 37 °C for 30 min.
3. Dialysis: the conjugate of the second antibody, the second single stranded DNA and the acceptor fluorescent molecule is sucked out from the EP tube and added to a dialysis bag (with a specification of 100 kd), which is tied and placed into a beaker containing 2 to 3 L TE solution for dialysis (the dialysis bag is soaked in advance). The dialysate is changed every 2 to 3 hours, and dialysis is performed for three times. After the dialysis is completed, the liquid in the dialysis bag is collected into a centrifuge tube and stored at 2 to 8°C for later use.

### Example 1

In this example, cardiac troponin I (cTnI) in whole blood is detected based on homogeneous immunoassay method of graphene oxide-antioxidant quenching and acridinium ester chemiluminescence. The first antibody is purchased from Hytest, with a clone number of 7b9cc, and the second antibody is purchased from Hytest, with a clone number of RecChim20C6. The two antibodies are coupled to the corresponding DNA molecule through the above preparation examples. The specific detection method includes the steps as follows:
1. Preparation of a detection reagent solution: a conjugate of a first single stranded DNA and a first antibody (DNA1-antibody 1 conjugate), a conjugate of a second antibody, a second single stranded DNA and quantum dots (antibody 2-DNA2-QDs conjugate), a conjugate of an acridinium ester and a third single stranded DNA, and an antioxidant-modified graphene oxide (GO-AOD) are mixed, so that their final concentrations are 10 nM, 10 nM, 0.15 µM and 20 µg/ml, respectively.
2. 50 µL of calibration solution with different concentrations or whole blood samples containing troponin I are mixed with 200 µL detection reagent solution, and incubated at 37 °C for 5 to 10 minutes.
3. After incubation, 200 µL of chemiluminescent substrate (an alkaline solution of hydrogen peroxide, wherien the concentration of hydrogen peroxide is 0.1 M) is added through a HSCL-10000 chemiluminescence instrument, and the chemiluminescent signal of the solution is immediately detected by a photomultiplier tube (PMT) for 3 s. The calibration curve of cardiac troponin (cTnI) and the concentration of cTnI in the whole blood sample to be measured are obtained according to the recorded chemiluminescence values (RLU).

The detection limit of cardiac troponin in this example ranges from 0.01 to 50 ng/mL after multiple detections.

40 clinical samples are detected by the method in this example and the Abbott detection method. The results show that the error between the detection value of cardiac troponin and the Abbott detection value in this example is -2.66%. The detection results are shown in Table 1 below, which shows that the detection method in this example has high accuracy. Abbott detection refers to detect the concentration of cTnI by using Abbott AXSYM immunofluorescence analyzer.

**Table 1 Comparison table of detection values in this example and Abbott detection method.**

| Number of cTnI samples | Abbott detection results (ng/mL) | This example (ng/mL) | Deviation | Number of cTnI samples | Abbott detection results (ng/mL) | This example (ng/mL) | Deviation |
|---|---|---|---|---|---|---|---|
| 1 | 0.08 | 0.09 | 12.5% | 21 | 30.2 | 26.65 | -11.75% |
| 2 | 0.1 | 0.1 | 0.00% | 22 | 43.4 | 49.55 | 14.17% |
| 3 | 0.05 | 0.05 | 0.00% | 23 | 48.41 | 41.73 | -13.80% |
| 4 | 0.05 | 0.04 | -20.0% | 24 | 6.29 | 5.81 | -7.63% |
| 5 | 0.04 | 0.04 | 0.00% | 25 | 12.96 | 13.85 | 6.87% |
| 6 | 0.07 | 0.07 | 0.00% | 26 | 11.97 | 10.58 | -11.61% |
| 7 | 0.08 | 0.07 | -12.50% | 27 | 38.99 | 36.67 | -5.95% |
| 8 | 0.01 | 0.01 | 0.00% | 28 | 27.57 | 28.76 | 4.32% |
| 9 | 0.02 | 0.02 | 0.00% | 29 | 6.29 | 7.02 | 11.61% |
| 10 | 0.06 | 0.06 | 0.00% | 30 | 13.26 | 14.44 | 8.90% |
| 11 | 0.62 | 0.58 | -6.45% | 31 | 34.73 | 31.24 | -10.05% |
| 12 | 0.77 | 0.67 | -12.99% | 32 | 10.28 | 9.59 | -6.71% |
| 13 | 0.36 | 0.31 | -13.89% | 33 | 0.12 | 0.12 | 0.00% |
| 14 | 0.42 | 0.42 | 0.00% | 34 | 29.7 | 32.04 | 7.88% |
| 15 | 0.78 | 0.68 | -12.82% | 35 | 39.58 | 36.75 | -7.15% |
| 16 | 0.98 | 0.98 | 0.00% | 36 | 28.28 | 26.65 | -5.76% |
| 17 | 0.3 | 0.34 | 13.33% | 37 | 48.71 | 43.32 | -11.07% |
| 18 | 8.2 | 7.87 | -4.02% | 38 | 7.35 | 7.47 | 1.63% |
| 19 | 22.93 | 19.61 | -14.48% | 39 | 35.66 | 35.66 | 10.10% |
| 20 | 22 | 19.18 | -12.82% | 40 | 41.81 | 41.81 | 3.85% |
| Total error | | | | | | | -2.66% |

### Example 2

In this example, procalcitonin (PCT) in whole blood is detected based on homogeneous immunoassay method of graphene oxide-antioxidant quenching and acridinium ester chemiluminescence. The first antibody is purchased from Chongqing Tansheng Technology Co., Ltd., with a clone number of 4A1, and the second antibody is purchased from Chongqing Tansheng Technology Co., Ltd., with a clone number of 10D6. The two antibodies are coupled to the corresponding DNA molecule through the above preparation examples. The specific detection method includes the steps as follows:
1. Preparation of a detection reagent solution: a conjugate of a first single stranded DNA and a first antibody, a conjugate of a second antibody, a second single stranded DNA and quantum dots, a conjugate of an acridinium ester and a third single stranded DNA, and an antioxidant-modified graphene oxide are mixed, so that their final concentrations are 10 nM, 10 nM, 0.15 µM, and 20 µg/ml, respectively.
2. 50 µL of calibration solution with different concentrations or whole blood samples containing procalcitonin are mixed with 200 µL detection reagent solution, and incubated at 37 °C for 5 to 10 minutes.
3. After incubation, 200 µL of chemiluminescent substrate (an alkaline solution of hydrogen peroxide, wherien the concentration of hydrogen peroxide is 0.1 M) is added through a HSCL-10000 chemiluminescence instrument, and the chemiluminescent signal of the solution is immediately detected by a photomultiplier tube (PMT) for 3 s. The calibration curve of PCT and the concentration of PCT in the whole blood sample to be measured are obtained according to the recorded chemiluminescence values (RLU).

The detection limit of PCT in this example ranges from 0.02 to 100 ng/mL after multiple detections. After detecting 40 clinical samples, the error between the detection value of PCT and the Roche detection value in this example is -2.24%, indicating that the detection method in this example has high accuracy. The detection results are shown in Table 2. Roche detection refers to Roche diagnostic procalcitonin (PCT) detection test kit, which is detected by using a Elecsys 2010 analyzer.

**Table 2 Comparison table of detection values in this example and Roche detection method**

| PCT Samples | Roche (ng/mL) | This example (ng/mL) | Deviation | PCT Samples | Roche (ng/mL) | This example (ng/mL) | Deviation |
|---|---|---|---|---|---|---|---|
| 1 | 0.032 | 0.031 | -3.13% | 21 | 95.82 | 97.679 | 1.94% |
| 2 | 0.03 | 0.027 | -10.00% | 22 | 43.4 | 84.03 | 3.32% |
| 3 | 0.033 | 0.033 | 0.00% | 23 | 81.33 | 96.717 | 6.61% |
| 4 | 0.028 | 0.027 | -3.57% | 24 | 90.72 | 41.769 | 9.00% |
| 5 | 0.03 | 0.03 | 0.00% | 25 | 38.32 | 28.277 | 0.31% |
| 6 | 0.029 | 0.029 | 0.00% | 26 | 28.19 | 25.181 | -12.17% |
| 7 | 0.028 | 0.028 | 0.00% | 27 | 28.67 | 70.718 | 3.92% |
| 8 | 0.04 | 0.044 | 10.00% | 28 | 68.05 | 26.4 | -0.79% |
| 9 | 0.034 | 0.029 | -14.71% | 29 | 26.61 | 48.79 | -4.37% |
| 10 | 0.047 | 0.042 | -10.64% | 30 | 80.77 | 69.276 | -14.23% |
| 11 | 1.732 | 1.588 | -8.31% | 31 | 21.41 | 19.102 | -10.78% |
| 12 | 0.434 | 0.495 | 14.06% | 32 | 76.35 | 81.206 | 6.36% |
| 13 | 1.268 | 1.41 | 11.20% | 33 | 38.28 | 34.628 | -9.54% |
| 14 | 0.253 | 0.221 | -12.65% | 34 | 56.59 | 51.723 | -8.60% |
| 15 | 1.858 | 1.971 | 6.08% | 35 | 91.34 | 85.138 | -6.79% |
| 16 | 0.055 | 0.055 | 0.00% | 36 | 41.07 | 39.452 | -3.94% |
| 17 | 0.34 | 0.382 | 12.35% | 37 | 79.13 | 68.02 | -14.04% |
| 18 | 65.71 | 66.085 | 0.57% | 38 | 35.23 | 34.585 | -1.83% |
| 19 | 32.68 | 28.193 | -13.73% | 39 | 90.82 | 78.323 | -13.76% |
| 20 | 45.15 | 49.62 | 9.90% | 40 | 48.891 | 45.168 | -7.65% |
| Total error | | | | | | | -2.24% |

### Example 3

In this example, thyroid stimulating hormone (TSH) in serum is detected based on homogeneous immunoassay method of graphene oxide-antioxidant quenching and acridinium ester chemiluminescence. The first antibody is purchased from Bionventix, with a clone number of 6C10; and the second antibody is purchased from Medix, with a clone number of 5409. The two antibodies are coupled through the above preparation examples. The specific detection method includes the steps as follows:
1. Preparation of a detection reagent solution: a conjugate of a first single stranded DNA and a first antibody, a conjugate of a second antibody, a second single stranded DNA and quantum dots, a conjugate of an acridinium ester and a third single stranded DNA, and an antioxidant-modified graphene oxide are mixed, so that their final concentrations are 10 nM, 10 nM, 0.15 µM, and 20 µg/ml, respectively.
2. 50 µL of calibration solution with different concentrations or serum samples containing thyroid stimulating hormone are mixed with 200 µL detection reagent solution, and incubated at 37 °C for 5 to 10 minutes.
3. After incubation, 200 µL of chemiluminescent substrate (an alkaline solution of hydrogen peroxide, wherien the concentration of hydrogen peroxide is 0.1 M) is added through a HSCL-10000 chemiluminescence instrument, and the chemiluminescent signal of the solution is immediately detected by a photomultiplier tube (PMT) for 3 s. The calibration curve of TSH and the concentration of TSH in the serum sample to be measured are obtained according to the recorded chemiluminescence values (RLU).

The detection limit of TSH in this example ranges from 0.005to 100 uIU/mL after multiple detections.

After detecting 40 clinical samples, the error between the detection value of TSH and the Roche detection value in this example is -3.37%, indicating that the detection method in this example has high accuracy. The detection results are shown in Table 3. Roche detection refers to conducting detection by using Roche TSH diagnostic kit (electrochemiluminescence method).

**Table 3 Comparison table of detection values in this example and Roche detection method**

| TSH Samples | Roche (ng/mL) | This example (ng/mL) | Deviation | TSH Samples | Roche (ng/mL) | This example (ng/mL) | Deviation |
|---|---|---|---|---|---|---|---|
| 1 | 1.284 | 1.231 | -4.13% | 21 | 95.82 | 63.817 | -6.70% |
| 2 | 0.617 | 0.547 | -11.35% | 22 | 68.4 | 13.68 | 6.05% |
| 3 | 1.619 | 1.377 | -14.95% | 23 | 99.66 | 91.637 | -8.05% |
| 4 | 1.848 | 1.792 | -3.03% | 24 | 95.44 | 86.988 | -8.86% |
| 5 | 0.668 | 0.619 | -7.34% | 25 | 25.68 | 23.492 | -8.52% |
| 6 | 1.016 | 0.997 | -1.87% | 26 | 22.76 | 19.915 | -12.50% |
| 7 | 0.843 | 0.749 | -11.15% | 27 | 66.68 | 60.532 | -9.22% |
| 8 | 0.574 | 0.552 | -3.83% | 28 | 76.1 | 75.446 | -0.86% |
| 9 | 1.423 | 1.315 | -7.59% | 29 | 84.45 | 95.699 | 13.32% |
| 10 | 9.729 | 8.652 | -11.07% | 30 | 78.82 | 73.925 | -6.21% |
| 11 | 6.675 | 6.663 | -0.18% | 31 | 30.01 | 30.589 | 1.93% |
| 12 | 2.334 | 2.482 | 6.34% | 32 | 10.14 | 11.34 | 11.83% |
| 13 | 2.414 | 2.703 | 11.97% | 33 | 64.84 | 62.5128 | -3.59% |
| 14 | 7.892 | 7.309 | -7.39% | 34 | 59.82 | 52.961 | -11.49% |
| 15 | 2.76 | 3.045 | 10.33% | 35 | 52.65 | 52.93 | 0.59% |
| 16 | 8.935 | 7.765 | -13.09% | 36 | 84.54 | 94.93 | 12.29% |
| 17 | 7.423 | 7.206 | -2.92% | 37 | 28.19 | 31.029 | 10.07% |
| 18 | 92.58 | 80.702 | -12.83% | 38 | 17.39 | 14.842 | -14.65% |
| 19 | 22.89 | 23.68 | 3.45% | 39 | 91.1 | 78.31 | -14.04% |
| 20 | 72.31 | 66.048 | -8.66% | 40 | 66.61 | 68.622 | 3.02% |
| Total error | | | | | | | -3.37% |

The present disclosure has been described by the above-mentioned related embodiments, however, the above-mentioned embodiments are merely examples for implementing the present disclosure. It must be noted that the disclosed embodiments do not limit the scope of the present disclosure. On the contrary, modifications and equivalents included in the spirit and scope of the claims are included within the scope of the present disclosure.

## Claims

1. A reagent composition for detecting a target protein, **characterized in that** the reagent composition for detecting a target protein comprises:
a first detection probe formed by coupling at least a first single stranded DNA and a first antibody; the first single stranded DNA comprises a first pairing sequence and a second pairing sequence; and the first antibody is capable of specifically binding to a first epitope of the target protein;
a second detection probe formed by coupling at least a second antibody, a second single stranded DNA, and an acceptor fluorescent molecule in sequence; the second single stranded DNA has a third pairing sequence and a fourth pairing sequence, the third pairing sequence is complementary to the second pairing sequence; and the second antibody is capable of specifically binding to a second epitope of the target protein;
a third detection probe formed by coupling at least a donor fluorescent molecule and a third single stranded DNA; the third single stranded DNA comprises a fifth pairing sequence and a sixth pairing sequence, the fifth pairing sequence is complementary to the fourth pairing sequence, the sixth pairing sequence is complementary to the first pairing sequence; the donor fluorescent molecule emiting a first fluorescence under a condition that it is capable of being oxidized by an oxidant; and the first fluorescence excits the acceptor fluorescent molecule to emit a second fluorescence under a condition that the first single stranded DNA, the second single stranded DNA, and the third single stranded DNA are paired with each other so as to obtain a content of the target protein according to an intensity of the second fluorescence; and
a fourth detection probe comprising an antioxidant for inhibiting the donor fluorescent molecule from emitting the first fluorescence.

2. The reagent composition for detecting a target protein according to claim 1, **characterized in that** the donor fluorescent molecule is an acridinium ester, and the acceptor fluorescent molecule is a quantum dot; the first single stranded DNA, the second single stranded DNA, and the third single stranded DNA are complementary and paired in the presence of the target protein to form a stem-loop structure, so that a distance between the donor fluorescent molecule and the acceptor fluorescent molecule is less than a limit distance at which fluorescence resonance energy transfer occur.

3. The reagent composition for detecting a target protein according to claim 2, **characterized in that**
the acridinium ester has a maximum emission wavelength of 430 nm; and
the quantum dot has a maximum absorption wavelength of 470 nm and a maximum emission wavelength of 605 nm; the quantum dot is a core-shell quantum dot having a core layer material selected from one or more of CdSe, CdS, CdTe, CdSeTe, CdZnS, ZnTe, CdSeS, PbS, and PbTe, and a shell layer material selected from one or more of ZnS, ZnSe, ZnSeS, PbS, and PbSeS.

4. The reagent composition for detecting a target protein according to any one of claims 1 to 3, **characterized in that** the first pairing sequence is ᵢₛₒGᵢₛₒCTᵢₛₒGAᵢₛₒGTT in an orientation from 5' end to 3' end, and the sixth pairing sequence is AAᵢₛₒCTᵢₛₒCAᵢₛₒGᵢₛₒC in an orientation from 5' end to 3' end;
ᵢₛₒG has a structural formula of: 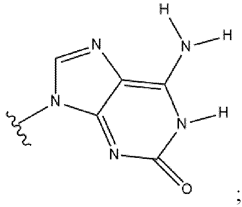 and
ᵢₛₒC has a structural formula of:

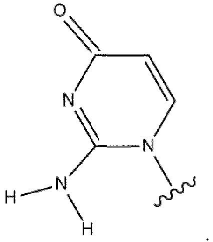

5. The reagent composition for detecting a target protein according to any one of claims 1 to 3, **characterized in that** the second pairing sequence is ᵢₛₒCAAᵢₛₒCᵢₛₒGAᵢₛₒC in an orientation from 5' end to 3' end, and the third pairing sequence is ᵢₛₒGTᵢₛₒCᵢₛₒGTTᵢₛₒG in an orientation from 5' end to 3' end;
ᵢₛₒG has a structural formula of: 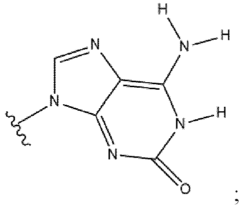 and
ᵢₛₒC has a structural formula of:

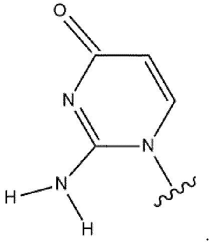

6. The reagent composition for detecting a target protein according to any one of claims 1 to 3, **characterized in that** the fourth pairing sequence is ᵢₛₒGᵢₛₒCTᵢₛₒGAᵢₛₒGAT in an orientation from 5' end to 3' end, and the fifth pairing sequence is ATᵢₛₒCTᵢₛₒCAᵢₛₒGᵢₛₒC in an orientation from 5' end to 3' end;
ᵢₛₒG has a structural formula of: 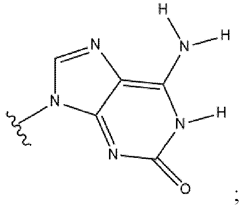 and
ᵢₛₒC has a structural formula of:

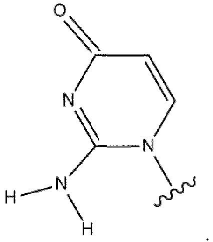

7. The reagent composition for detecting a target protein according to any one of claims 1 to 3, **characterized in that** a full-length sequence of the first single stranded DNA is a sequence in which at least a part or all of G in a sequence shown in SEQ ID No: 1 is replaced by ᵢₛₒG and at least a part or all of C is replaced by ᵢₛₒC; a full-length sequence of the second single stranded DNA is a sequence in which at least a part or all of G in a sequence shown in SEQ ID No: 2 is replaced by ᵢₛₒG and at least a part or all of C is replaced by ᵢₛₒC; and
a full-length sequence of the third single stranded DNA is a sequence in which at least a part or all of G in a sequence shown in SEQ ID No: 3 is replaced by ᵢₛₒG and at least a part or all of C is replaced by ᵢₛₒC;
wherein ᵢₛₒG has a structural formula of: 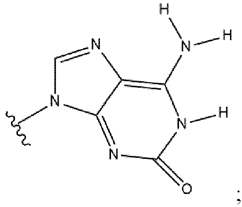 and
ᵢₛₒC has a structural formula of:

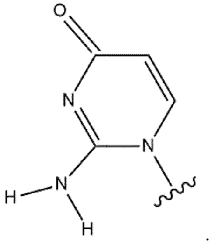

8. The reagent composition for detecting a target protein according to any one of claims 4 to 7, **characterized in that**
a bonding form of ᵢₛₒG and ᵢₛₒC is as follows: 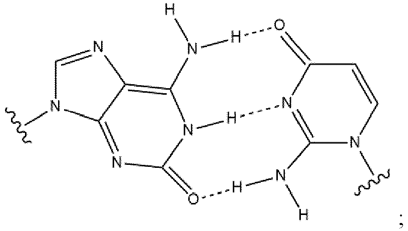 wherein 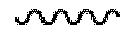 indicates a site connecting to deoxyribose in a DNA molecule.

9. The reagent composition for detecting a target protein according to any one of claims 1 to 8, **characterized in that** the fourth detection probe further comprises a carrier molecule whose surface binds to the antioxidant;
the antioxidant is selected from any one or more of cannabidiol, vitamin C, vitamin E, tea polyphenol and glutathione; and
the oxidant comprises an alkaline solution of hydrogen peroxide.

10. The reagent composition for detecting a target protein according to claim 9, **characterized in that** the carrier molecule is graphene oxide; a carboxyl group on the graphene oxide is bonded to a hydroxyl group on the antioxidant through a sulfoxide condensing agent, and the carboxyl group on the graphene oxide is bonded to an amino group on the antioxidant through 1-(3-dimethylaminopropyl)-3-ethylcarbodiimide hydrochloride.

11. A method for detecting a target protein, **characterized in that** the method for detecting a target protein comprises:
adding a first detection probe, a second detection probe, a third detection probe and a fourth detection probe into a solution to be detected, and performing mixing to form a sample to be detected; the first detection probe is formed by coupling at least a first single stranded DNA and a first antibody, the second detection probe is formed by coupling at least a second antibody, a second single stranded DNA, and an acceptor fluorescent molecule in sequence, and the third detection probe is formed by coupling at least a donor fluorescent molecule and a third single stranded DNA; and under a condition that the target protein is contained in a solution to be detected, the first antibody, the target protein and the second antibody form a double-antibody sandwich structure, the first single stranded DNA, the second single stranded DNA and the third single stranded DNA form a stem-loop structure, and the donor fluorescent molecule and the acceptor fluorescent molecule are located on a same side of the stem-loop structure;
removing the fourth detection probe for inhibiting oxidation of the donor fluorescent molecule from the sample to be detected, adding an oxidant for oxidizing the donor fluorescent molecule to emit a first fluorescence, and collecting a second fluorescence according to a maximum emission wavelength of the acceptor fluorescent molecule; and
determining that the target protein is not contained in the solution to be detected in a case that the second fluorescence is not collected; and obtaining a content of the target protein in the solution to be detected according to an intensity of the second fluorescence based on a functional relationship between fluorescence intensity of and protein content in a case that the second fluorescence is collected.

12. The method for detecting a target protein according to claim 11, **characterized in that** a working concentration of the first detection probe in the sample to be detected ranges from 1 nM to 20 nM; a working concentration of the second detection probe in the sample to be detected ranges from 1 nM to 20 nM;
a working concentration of the third detection probe in the sample to be detected ranges from 0.05 nM to 0.2 nM; and
a working concentration of the fourth detection probe in the sample to be detected ranges from 15 µg/ml to 25 µg/ml.

13. The method for detecting a target protein according to claim 11, **characterized in that** the solution to be detected is from a whole blood sample, a serum sample, or a plasma sample.

14. The method for detecting a target protein according to claim 11, **characterized in that** the target protein comprises troponin, procalcitonin, or thyroid stimulating hormone.

15. A kit for detecting a target protein, **characterized in that** the kit for detecting a target protein comprises:
a first container for storing at least a conjugate of a first single stranded DNA and a first antibody;
a second container for storing at least a conjugate of a second antibody, a second single stranded DNA, and an acceptor fluorescent molecule; the first antibody and the second antibody are capable of forming a double-antibody sandwich structure with the target protein under a condition that the target protein is present;
a third container for storing at least a conjugate of a donor fluorescent molecule and a third single stranded DNA; the first single stranded DNA, the second single stranded DNA, and the third single stranded DNA are capable of forming a stem-loop structure under a condition that the double-antibody sandwich structure is formed, and the acceptor fluorescent molecule is excited to emit a second fluorescence so as to obtain a content of the target protein according to an intensity of the second fluorescence in a case that the donor fluorescent molecule and the acceptor fluorescent molecule are located on a same side of the stem-loop structure;
a fourth container for storing at least an antioxidant capable of inhibiting oxidation of the donor fluorescent molecule; and
a fifth container for storing at least an oxidant capable of oxidizing the donor fluorescent molecule to emit a first fluorescence.

16. The kit for detecting a target protein according to claim 15, **characterized in that** the donor fluorescent molecule is an acridinium ester; and the acceptor fluorescent molecule is a quantum dot.

17. The kit for detecting a target protein according to claim 15, **characterized in that** the acridinium ester has a maximum emission wavelength of 430 nm; and the quantum dot has a maximum absorption wavelength of 470 nm and a maximum emission wavelength of 605 nm.

18. The kit for detecting a target protein according to claim 15, **characterized in that** the first single stranded DNA comprises a first pairing sequence and a second pairing sequence; and the first antibody is capable of specifically binding to a first epitope of the target protein;
the second single stranded DNA has a third pairing sequence and a fourth pairing sequence, the third pairing sequence is complementary to the second pairing sequence; and the second antibody is capable of specifically binding to a second epitope of the target protein;
the third single stranded DNA comprises a fifth pairing sequence and a sixth pairing sequence, the fifth pairing sequence is complementary to the fourth pairing sequence, and the sixth pairing sequence is complementary to the first pairing sequence;
at least a part or all of the first pairing sequence, the second pairing sequence, the third pairing sequence, the fourth pairing sequence, the fifth pairing sequence, and the sixth pairing sequence contain ᵢₛₒG and ᵢₛₒC;
ᵢₛₒG has a structural formula of: 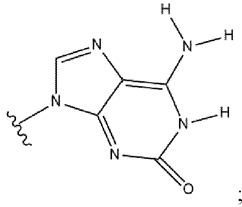 and
ᵢₛₒC has a structural formula of:

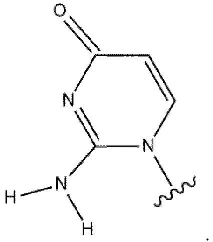

19. A system for detecting a target protein, **characterized in that** the system for detecting a target protein comprises:
a reaction vessel comprising an accommodating chamber capable of accommodating a solution to be detected;
a microinjection pump communicated with the accommodating chamber through an injection pipeline for injecting a mixture of a first detection probe, a second detection probe, a third detection probe, and a fourth detection probe into the accommodating chamber; the first detection probe is formed by coupling at least a first single stranded DNA and a first antibody, the second detection probe is formed by coupling at least a second antibody, a second single stranded DNA, and an acceptor fluorescent molecule in sequence, the third detection probe is formed by coupling at least a donor fluorescent molecule and a third single stranded DNA, and the fourth detection probe comprises an antioxidant for inhibiting the donor fluorescent molecule from emitting a first fluorescence;
a filter disposed on an emergent light path of the first fluorescence, allowing a second fluorescence having a same wavelength as a maximum emission wavelength of the acceptor fluorescent molecule to pass through;
an optical signal detection module disposed on an emergent light path of the first fluorescence and located on a downstream side of the filter to acquire the second fluorescence transmitted from the filter; and
a calculation module for converting the second fluorescence into a digital signal and obtaining a content of the target protein in the solution to be detected based on a functional relationship between fluorescence intensity and protein content.
